# EUROPEAN PATENT SPECIFICATION

(11) **EP 0 999 848 B1**
(45) Date of publication and mention of the grant of the patent: **21.02.2007**
(21) Application number: 98966066.7
(22) Date of filing: 23.12.1998
(51) Int. Cl.: A61K 38/16, A61K 38/28, A61K 39/00, C07K 14/00, C07K 14/435, C07K 14/62, C12N 9/88

(54) **CHIMERIC PROTEINS FOR THE TREATMENT OF DIABETES**
CHIMÄRE PROTEINE ZUR BEHANDLUNG VON DIABETES
PROTEINES CHIMERES POUR LE TRAITEMENT DU DIABETE

(30) Priority: 23.12.1997 US 68648 P
(43) Date of publication of application: 17.05.2000
(73) Proprietor: ALEXION PHARMACEUTICALS, INC., New Haven, CT 06511 (US)
(72) Inventor: MUELLER, John, P., Old Lyme, CT 06371 (US); MATIS, Louis, A., Southport, CT 06490 (US); WANG, Yi, Orange, CT 06477 (US)
(74) Representative: Maschio, Antonio
(86) International application number: PCT/US1998/027408
(87) International publication number: WO 1999/032136

(56) References cited:
- EP-A- 0 940 470
- WO-A-96/26218
- US-A- 4 431 740
- US-A- 4 939 094
- US-A- 5 691 448
- US-A- 5 821 334
- RAMIYA et al., "Immunization Therapies in the Prevention of Diabetes", JOURNAL OF AUTOIMMUNITY, 1997, Vol. 10, pages 287-292, XP002918457

### SEQUENCE LISTING

<110> Wang, Yi
   Mueller, John P.
   Matis, Louis A.
<120> Chimeric Protiens and uses thereof for the Diagnosis,
   Prevention, and Treatment of Diabetes
<130> ALX-156 PCT
<140> Not Yet Assigned
   <141> 1998-12-18
<150> 60/068,648
   <151> 1997-12-22
<160> 37
<170> PatentIn Ver. 2.0
<210> 1
   <211> 160
   <212> PRT
   <213> Artificial Sequence
<220>
   <223> Description of Artificial Sequence:IG1 Fusion
   Protein
<400> 1
   Met Phe Val Asn Gln His Leu Cys Gly Ser His Leu Val Glu Ala Leu
    1               5                  10                  15
   Tyr Leu Val Cys Gly Glu Arg Gly Phe Phe Tyr Thr Pro Lys Thr Arg
               20                  25                  30
   Arg Glu Ala Glu Asp Leu Asn Met Tyr Ala Met Met Ile Ala Arg Phe
           35                  40                  45
   Lys Met Phe Pro Glu Val Lys Glu Lys Gly Met Ala Ala Leu Pro Arg
       50                  55                  60
   Leu Ile Ala Phe Thr Ser Glu Lys Cys Leu Glu Leu Ala Glu Tyr Leu
   65                  70                  75                  80
   Tyr Asn Ile Ile Lys Asn Arg Glu Gly Tyr Glu Met Val Phe Asp Gly
                   85                  90                  95
   Lys Pro Gln His Thr Asn Val Cys Phe Trp Tyr Ile Pro Pro Ser Leu
               100                 105                 110
   Arg Thr Leu Glu Asp Asn Glu Glu Arg Met Ser Arg Leu Ser Lys Val
           115                 120                 125
   Ala Pro Val Ile Lys Ala Arg Met Met Glu Tyr Gly Thr Thr Met Val
       130                 135                 140
   Ser Tyr Gln Pro Leu Gly Asp Lys Val Asn His His His His His His
   145                 150                 155                 160
<210> 2
   <211> 180
   <212> PRT
   <213> Artificial Sequence
<220>
   <223> Description of Artificial Sequence:IG2 Fusion
   Protein
<400> 2
   Met Phe Val Asn Gln His Leu Cys Gly Ser His Leu Val Glu Ala Leu
    1               5                  10                  15
   Tyr Leu Val Cys Gly Glu Arg Gly Phe Phe Tyr Thr Pro Lys Thr Arg
               20                  25                  30
   Arg Glu Ala Glu Asp Leu Met Asn Ile Leu Leu Gln Tyr Val Val Lys
           35                  40                  45
   Ser Phe Asp Asn Met Tyr Ala Met Met Ile Ala Arg Phe Lys Met Phe
       50                  55                  60
   Pro Glu Val Lys Glu Lys Gly Met Ala Ala Leu Pro Arg Leu Ile Ala
   65 70 75 80
   Phe Thr Ser Glu His Ser His Phe Ser Leu Lys Lys Cys Leu Glu Leu
   85 90 95
   Ala Glu Tyr Leu Tyr Asn Ile Ile Lys Asn Arg Glu Gly Tyr Glu Met
   100 105 110
   Val Phe Asp Gly Lys Pro Gln His Thr Asn Val Cys Phe Trp Tyr Ile
   115 120 125
   Pro Pro Ser Leu Arg Thr Leu Glu Asp Asn Glu Glu Arg Met Ser Arg
   130 135 140
   Leu Ser Lys Val Ala Pro Val Ile Lys Ala Arg Met Met Glu Tyr Gly
   145 150 155 160
   Thr Thr Met Val Ser Tyr Gln Pro Leu Gly Asp Lys Val Asn His His
   165 170 175
   His His His His
   180
<210> 3
   <211> 144
   <212> PRT
   <213> Artificial Sequence
<220>
   <223> Description of Artificial Sequence:IG3 Fusion
   Protein
   <400> 3
   Met Phe Val Asn Gln His Leu Cys Gly Ser His Leu Val Glu Ala Leu
   1 5 10 15
   Tyr Leu Val Cys Gly Glu Arg Gly Phe Phe Tyr Thr Pro Lys Thr Arg
   20 25 30
   Arg Glu Ala Glu Asp Leu Met Asn Ile Leu Leu Gln Tyr Val Val Lys
   35 40 45
   Ser Phe Asp Asn Met Tyr Ala Met Met Ile Ala Arg Phe Lys Met Phe
   50 55 60
   Pro Glu Val Lys Glu Lys Gly Met Ala Ala Leu Pro Arg Leu Ile Ala
   65 70 75 80
   Phe Thr Ser Glu His Ser His Phe Ser Leu Lys Lys Cys Leu Glu Leu
   85 90 95
   Ala Glu Tyr Leu Tyr Asn Ile Ile Lys Asn Arg Glu Gly Tyr Glu Met
   100 105 110
   Val Phe Asp Gly Lys Pro Gln His Thr Asn Val Cys Phe Trp Tyr Ile
   115 120 125
   Pro Pro Ser Leu Arg Thr Leu Glu Asp Asn His His His His His His
   130 135 140
<210> 4
   <211> 181
   <212> PRT
   <213> Artificial Sequence
<220>
   <223> Description of Artificial Sequence:IG4 Fusion
   Protein
<400> 4
   Met Phe Val Asn Gln His Leu Cys Gly Ser His Leu Val Glu Ala Leu
   1 5 10 15
   Tyr Leu Val Cys Gly Glu Arg Gly Phe Phe Tyr Thr Pro Lys Thr Arg
   20 25 30
   Arg Glu Ala Glu Asp Leu Gln Val Gly Gln Val Glu Leu Gly Gly Gly
   35 40 45
   Pro Gly Ala Gly Ser Leu Gln Pro Leu Ala Leu Glu Gly Ser Leu Gln
   50 55 60
   Lys Arg Gly Thr Asn Met Phe Thr Tyr Glu Ile Ala Pro Val Phe Val
   65 70 75 80
   Leu Leu Glu Tyr Val Thr Leu Lys Lys Met Arg Glu Ile Ile Gly Trp
   85 90 95
   Pro Gly Gly Ser Gly Asp Gly Gly Gly Met Asn Ile Leu Leu Gln Tyr
   100 105 110
   Val Val Lys Ser Phe Asp Asn Met Tyr Ala Met Met Ile Ala Arg Phe
   115 120 125
   Lys Met Phe Pro Glu Val Lys Glu Lys Gly Met Ala Ala Leu Pro Arg
   130 135 140
   Leu Gly Gly Gly Ile Ala Phe Thr Ser Glu His Ser His Phe Ser Leu
   145 150 155 160
   Lys Lys Gly Ala Ala Ala Leu Gly Ile Gly Thr Asp Ser Val Ile His
   165 170 175
   His His His His His
   180
<210> 5
   <211> 232
   <212> PRT
   <213> Artificial Sequence
<220>
   <223> Description of Artificial Sequence:IG5 Fusion
   Protein
<400> 5
   Met Phe Val Asn Gln His Leu Cys Gly Ser His Leu Val Glu Ala Leu
   1 5 10 15
   Tyr Leu Val Cys Gly Glu Arg Gly Phe Phe Tyr Thr Pro Lys Thr Arg
   20 25 30
   Arg Glu Ala Glu Asp Leu Gln Val Gly Gln Val Glu Leu Gly Gly Gly
   35 40 45
   Pro Gly Ala Gly Ser Leu Gln Pro Leu Ala Leu Glu Gly Ser Leu Gln
   50 55 60
   Lys Arg Gly Thr Asn Met Phe Thr Tyr Glu Ile Ala Pro Val Phe Val
   65 70 75 80
   Leu Leu Glu Tyr Val Thr Leu Lys Lys Met Arg Glu Ile Ile Gly Trp
   85 90 95
   Pro Gly Gly Ser Gly Asp Gly Gly Gly Met Asn Ile Leu Leu Gln Tyr
   100 105 110
   Val Val Lys Ser Phe Asp Asn Met Tyr Ala Met Met Ile Ala Arg Phe
   115 120 125
   Lys Met Phe Pro Glu Val Lys Glu Lys Gly Met Ala Ala Leu Pro Arg
   130 135 140
   Leu Gly Gly Gly Ile Ala Phe Thr Ser Glu His Ser His Phe Ser Leu
   145 150 155 160
   Lys Lys Gly Ala Ala Ala Leu Gly Ile Gly Thr Asp Ser Val Ile Gly
   165 170 175
   Gly Gly Tyr Ile Pro Pro Ser Leu Arg Thr Leu Glu Asp Asn Glu Glu
   180 185 190
   Arg Met Ser Arg Leu Ser Lys Val Ala Pro Val Ile Lys Ala Arg Met
   195 200 205
   Met Glu Tyr Gly Thr Thr Met Val Ser Tyr Gln Pro Leu Gly Asp Lys
   210 215 220
   Val Asn His His His His His His
   225 230
<210> 6
   <211> 393
   <212> PRT
   <213> Artificial Sequence
<220>
   <223> Description of Artificial Sequence:IG6 Fusion
   Protein
<400> 6
   Met Phe Val Asn Gln His Leu Cys Gly Ser His Leu Val Glu Ala Leu
   1               5                   10                  15
   Tyr Leu Val Cys Gly Glu Arg Gly Phe Phe Tyr Thr Pro Lys Thr Arg
               20                  25                  30
   Arg Glu Ala Glu Asp Leu Gln Val Gly Gln Val Glu Leu Gly Gly Gly
           35                  40                  45
   Pro Gly Ala Gly Ser Leu Gln Pro Leu Ala Leu Glu Gly Ser Leu Gln
       50                  55                  60
   Lys Arg Gly Thr Asn Met Phe Thr Tyr Glu Ile Ala Pro Val Phe Val
   65                  70                  75                  80
   Leu Leu Glu Tyr Val Thr Leu Lys Lys Met Arg Glu Ile Ile Gly Trp
                   85                  90                  95
   Pro Gly Gly Ser Gly Asp Gly Gly Gly Met Asn Ile Leu Leu Gln Tyr
               100                 105                 110
   Val Val Lys Ser Phe Asp Asn Met Tyr Ala Met Met Ile Ala Arg Phe
           115                 120                 125
   Lys Met Phe Pro Glu Val Lys Glu Lys Gly Met Ala Ala Leu Pro Arg
       130                 135                 140
   Leu Gly Gly Gly Ile Ala Phe Thr Ser Glu His Ser His Phe Ser Leu
   145                 150                 155                 160
   Lys Lys Gly Ala Ala Ala Leu Gly Ile Gly Thr Asp Ser Val Ile Gly
                   165                 170                 175
   Gly Gly Ile Glu His Asp Pro Arg Met Pro Ala Tyr Ile Ala Thr Gln
               180                 185                 190
   Gly Pro Leu Ser His Thr Ile Ala Asp Phe Trp Gln Met Val Trp Glu
           195                 200                 205
   Ser Gly Cys Thr Val Ile Val Met Leu Thr Pro Leu Val Glu Asp Gly
       210                 215                 220
   Val Lys Gln Cys Asp Arg Tyr Trp Pro Asp Glu Gly Ala Ser Leu Tyr
   225                 230                 235                 240
   His Val Tyr Glu Val Asn Leu Val Ser Glu His Ile Trp Cys Glu Asp
                   245                 250                 255
   Phe Leu Val Arg Ser Phe Tyr Leu Lys Asn Val Gln Thr Gln Glu Thr
               260                 265                 270
   Arg Thr Leu Thr Gln Phe His Phe Leu Ser Trp Pro Ala Glu Gly Thr
           275                 280                 285
   Pro Ala Ser Thr Arg Pro Leu Leu Asp Phe Arg Arg Lys Val Asn Lys
       290                 295                 300
   Cys Tyr Arg Gly Arg Ser Cys Pro Ile Ile Val His Cys Ser Asp Gly
   305                 310                 315                 320
   Ala Gly Arg Thr Gly Thr Tyr Ile Leu Ile Asp Met Val Leu Asn Arg
                   325                 330                 335
   Met Ala Lys Gly Val Lys Glu Ile Asp Ile Ala Ala Thr Leu Glu His
               340                 345                 350
   Val Arg Asp Gln Arg Pro Gly Leu Val Arg Ser Lys Asp Gln Phe Glu
           355                 360                 365
   Phe Ala Leu Thr Ala Val Ala Glu Glu Val Asn Ala Ile Leu Lys Ala
       370                 375                 380
   Leu Pro Gln His His His His His His
   385                 390
<210> 7
   <211> 444
   <212> PRT
   <213> Artificial Sequence
<220>
   <223> Description of Artificial Sequence:IG7 Fusion
   Protein
<400> 7
<210> 8
   <211> 173
   <212> PRT
   <213> Artificial Sequence
<220>
   <223> Description of Artificial Sequence:IG4NHB
   hypothetical fusion protein
<400> 8
   Met Phe Val Asn Gln His Leu Cys Gly Ser His Leu Val Glu Ala Leu
   1 5 10 15
   Tyr Leu Val Cys Gly Glu Arg Gly Phe Phe Tyr Thr Pro Lys Thr Arg
   20 25 30
   Arg Glu Ala Glu Asp Leu Gln Val Gly Gln Val Glu Leu Gly Gly Gly
   35 40 45
   Pro Gly Ala Gly Ser Leu Gln Pro Leu Ala Leu Glu Gly Ser Leu Gln
   50 55 60
   Lys Arg Gly Met Asn Ile Leu Leu Gln Tyr Val Val Lys Ser Phe Asp
   65 70 75 80
   Asn Met Tyr Ala Met Met Ile Ala Arg Phe Lys Met Phe Pro Glu Val
   85 90 95
   Lys Glu Lys Gly Met Ala Ala Leu Pro Arg Leu Ile Ala Phe Thr Ser
   100 105 110
   Glu His Ser His Phe Ser Leu Lys Lys Cys Leu Glu Leu Ala Glu Tyr
   115 120 125
   Leu Tyr Asn Ile Ile Lys Asn Arg Glu Gly Tyr Glu Met Val Phe Asp
   130 135 140
   Gly Lys Pro Gln His Thr Asn Val Cys Phe Trp Tyr Ile Pro Pro Ser
   145 150 155 160
   Leu Arg Thr Leu Glu Asp Asn His His His His His His
   165 170
<210> 9
   <211> 3
   <212> PRT
   <213> Artificial Sequence
<220>
   <223> Description of Artificial Sequence:Helix breaker
<400> 9
   Pro Pro Pro
   1
<210> 10
   <211> 3
   <212> PRT
   <213> Artificial Sequence
<220>
   <223> Description of Artificial Sequence:Helix breaker
<400> 10
   Gly Gly Gly
   1
<210> 11
   <211> 139
   <212> DNA
   <213> Artificial Sequence
<220>
   <223> Description of Artificial Sequence:prIG1 primer
<400> 11
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgaac 120
   atgtatgcca tgatgatcg                                              139
<210> 12
   <211> 143
   <212> DNA
   <213> Artificial Sequence
<220>
   <223> Description of Artificial Sequence:prIG2 primer
<400> 12
   ggtttttaat gatgttgtac agatattccg ccagttccag acatttttca gaggtaaagg 60
   caatcagacg cggcagcgcg gccatacctt tttctttaac ttccgggaac attttaaagc 120
   gcgcgatcat catggcatac atg                                         143
<211> 138
   <212> DNA
   <213> Artificial Sequence
<220>
   <223> Description of Artificial Sequence:prIG3 primer
<400> 13
<210> 14
   <211> 132
   <212> DNA
   <213> Artificial Sequence
<220>
   <223> Description of Artificial Sequence:prIG4 primer
<400> 14
   ggatccttaa tggtgatggt gatggtggtt aactttatca cccagcggct ggtagctaac 60
   catggtggtg ccatattcca tcatgcgcgc tttaataacc ggggcaactt tagacagacg 120
   gctcatgcgt tc                                                     132
<210> 15
   <211> 18
   <212> DNA
   <213> Artificial Sequence
<220>
   <223> Description of Artificial Sequence:prIG5 primer
<400> 15
   catatgttcg ttaaccag 18
<210> 16
   <211> 18
   <212> DNA
   <213> Artificial Sequence
<220>
   <223> Description of Artificial Sequence:prIG6 primer
<400> 16
   ggatccttaa tggtgatg 18
<210> 17
   <211> 492
   <212> DNA
   <213> Artificial Sequence
<220>
   <223> Description of Artificial Sequence:IG1 Fusion
   Protein coding sequence
<400> 17
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgaac 120
   atgtatgcca tgatgatcgc gcgctttaaa atgttcccgg aagttaaaga aaaaggtatg 180
   gccgcgctgc cgcgtctgat tgcctttacc tctgaaaaat gtctggaact ggcggaatat 240
   ctgtacaaca tcattaaaaa ccgcgaaggc tatgaaatgg ttttcgatgg taaaccgcag 300
   cataccaacg tttgcttttg gtacatcccg ccgagcctgc gtaccctgga agataacgaa 360
   gaacgcatga gccgtctgtc taaagttgcc ccggttatta aagcgcgcat gatggaatat 420
   ggcaccacca tggttagcta ccagccgctg ggtgataaag ttaaccacca tcaccatcac 480
   cattaaggat cc                                                     492
<210> 18
   <211> 64
   <212> DNA
   <213> Artificial Sequence
<220>
   <223> Description of Artificial Sequence:prIG7 primer
<400> 18
   agatctgatg aacattctgc tgcagtatgt tgttaaaagc ttcgataaca tgtatgccat 60
   gatg                                                              64
<210> 19
   <211> 78
   <212> DNA
   <213> Artificial Sequence
<220>
   <223> Description of Artificial Sequence:prIG8 primer
<400> 19
   tgtacagata ttccgccagt tccagacatt ttttcagaga aaaatggcta tgttcagagg 60
   taaaggcaat cagacgcg 78
<210> 20
   <211> 27
   <212> DNA
   <213> Artificial Sequence
<220>
   <223> Description of Artificial Sequence:prIG12 primer
<400> 20
   tgtacagata ttccgccagt tccagac 27
<210> 21
   <211> 552
   <212> DNA
   <213> Artificial Sequence
<220>
   <223> Description of Artificial Sequence:IG2 Fusion
   Protein coding sequence
<400> 21
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgatg 120
   aacattctgc tgcagtatgt tgttaaaagc ttcgataaca tgtatgccat gatgatcgcg 180
   cgctttaaaa tgttcccgga agttaaagaa aaaggtatgg ccgcgctgcc gcgtctgatt 240
   gcctttacct ctgaacatag ccatttttct ctgaaaaaat gtctggaact ggcggaatat 300
   ctgtacaaca tcattaaaaa ccgcgaaggc tatgaaatgg ttttcgatgg taaaccgcag 360
   cataccaacg tttgcttttg gtacatcccg ccgagcctgc gtaccctgga agataacgaa 420
   gaacgcatga gccgtctgtc taaagttgcc ccggttatta aagcgcgcat gatggaatat 480
   ggcaccacca tggttagcta ccagccgctg ggtgataaag ttaaccacca tcaccatcac 540
   cattaaggat cc 552
<210> 22
   <211> 46
   <212> DNA
   <213> Artificial Sequence
<220>
   <223> Description of Artificial Sequence:prIG13 primer
<400> 22
   ggatccttaa atggtgatgg tgatggtggt tatcttccag ggtacg 46
<210> 23
   <211> 444
   <212> DNA
   <213> Artificial Sequence
<220>
   <223> Description of Artificial Sequence:IG3 Fusion
   Protein coding sequence
<400> 23
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgatg 120
   aacattctgc tgcagtatgt tgttaaaagc ttcgataaca tgtatgccat gatgatcgcg 180
   cgctttaaaa tgttcccgga agttaaagaa aaaggtatgg ccgcgctgcc gcgtctgatt 240
   gcctttacct ctgaacatag ccatttttct ctgaaaaaat gtctggaact ggcggaatat 300
   ctgtacaaca tcattaaaaa ccgcgaaggc tatgaaatgg ttttcgatgg taaaccgcag 360
   cataccaacg tttgcttttg gtacatcccg ccgagcctgc gtaccctgga agataaccac 420
   catcaccatc accattaagg atcc 444
<210> 24
   <211> 555
   <212> DNA
   <213> Artificial Sequence
<220>
   <223> Description of Artificial Sequence:IG4 Fusion
   Protein coding sequence
<400> 24
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgcag 120
   gtggggcagg tggagctggg cgggggccct ggtgcaggca gcctgcagcc cttggccctg 180
   gaggggtccc tgcagaagcg tggcactaac atgttcacct atgaaattgc tccagtattt 240
   gtgcttttgg aatatgtcac actaaagaaa atgagagaaa tcattggctg gccagggggc 300
   tctggcgatg gaggcggtat gaacattctg ctgcagtatg ttgttaaaag cttcgataac 360
   atgtatgcca tgatgatcgc gcgctttaaa atgttcccgg aagttaaaga aaaaggtatg 420
   gccgcgctgc cgcgtctggg aggcggtatt gcctttacct ctgaacatag ccatttttct 480
   ctgaaaaaag gagctgcagc cttagggatt ggaacagaca gcgtgattca ccatcaccat 540
   caccattaag gatcc 555
<210> 25
   <211> 24
   <212> DNA
   <213> Artificial Sequence
<220>
   <223> Description of Artificial Sequence:prIG14 primer
<400> 25
   catatgttcg ttaaccagca tctg 24
<210> 26
   <211> 69
   <212> DNA
   <213> Artificial Sequence
<220>
   <223> Description of Artificial Sequence:prIG15 primer
<400> 26
   gctgcctgca ccagggcccc cgcccagctc cacctgcccc acctgcagat cttccgcttc 60
   acgacgggt 69
<210> 27
   <211> 66
   <212> DNA
   <213> Artificial Sequence
<220>
   <223> Description of Artificial Sequence:prIG16 primer
<400> 27
   agtgccacgc ttctgcaggg acccctccag ggccaagggc tgcaggctgc ctgcaccagg 60
   gccccc 66
<210> 28
   <211> 69
   <212> DNA
   <213> Artificial Sequence
<220>
   <223> Description of Artificial Sequence:prIG17 primer
<400> 28
   ttccaaaagc acaaatactg gagcaatttc ataggtgaac atgttagtgc cacgcttctg 60
   cagggaccc 69
<210> 29
   <211> 69
   <212> DNA
   <213> Artificial Sequence
<220>
   <223> Description of Artificial Sequence:prIG18 primer
<400> 29
   ccctggccag ccaatgattt ctctcatttt ctttagtgtg acatattcca aaagcacaaa 60
   tactggagc 69
<210> 30
   <211> 69
   <212> DNA
   <213> Artificial Sequence
<220>
   <223> Description of Artificial Sequence:prIG19 primer
<400> 30
   agagaaatca ttggctggcc agggggctct ggcgatggag gcggtatgaa cattctgctg 60
   cagtatgtt 69
<210> 31
   <211> 68
   <212> DNA
   <213> Artificial Sequence
<220>
   <223> Description of Artificial Sequence:prIG20 primer
<400> 31
   cagagaaaaa tggctatgtt cagaggtaaa ggcaataccg cctcccagac gcggcagcgc 60
   ggccatac 68
<210> 32
   <211> 69
   <212> DNA
   <213> Artificial Sequence
<220>
   <223> Description of Artificial Sequence:prIG21 primer
<400> 32
   aatcacgctg tctgttccaa tccctaaggc tgcagctcct tttttcagag aaaaatggct 60
   atgttcaga 69
<210> 33
   <211> 60
   <212> DNA
   <213> Artificial Sequence
<220>
   <223> Description of Artificial Sequence:prIG22 primer
<400> 33
   ttagggattg gaacagacag cgtgattgga ggcggttaca tcccgccgag cctgcgtacc 60
<210> 34
   <211> 24
   <212> DNA
   <213> Artificial Sequence
<220>
   <223> Description of Artificial Sequence:prIG23 primer
<400> 34
   ggatccttaa tggtgatggt gatg 24
<210> 35
   <211> 708
   <212> DNA
   <213> Artificial Sequence
<220>
   <223> Description of Artificial Sequence:IG5 Fusion
   Protein coding sequence
<400> 35
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgcag 120
   gtggggcagg tggagctggg cgggggccct ggtgcaggca gcctgcagcc cttggccctg 180
   gaggggtccc tgcagaagcg tggcactaac atgttcacct atgaaattgc tccagtattt 240
   gtgcttttgg aatatgtcac actaaagaaa atgagagaaa tcattggctg gccagggggc 300
   tctggcgatg gaggcggtat gaacattctg ctgcagtatg ttgttaaaag cttcgataac 360
   atgtatgcca tgatgatcgc gcgctttaaa atgttcccgg aagttaaaga aaaaggtatg 420
   gccgcgctgc cgcgtctggg aggcggtatt gcctttacct ctgaacatag ccatttttct 480
   ctgaaaaaag gagctgcagc cttagggatt ggaacagaca gcgtgattgg aggcggttac 540
   attcctccaa gcttgcgtac tctggaagac aatgaagaac gcatgagccg tctgtctaaa 600
   gttgccccgg ttattaaagc gcgcatgatg gaatatggca ccaccatggt tagctaccag 660
   ccgctgggtg ataaagttaa ccaccatcac catcaccatt aaggatcc 708
<210> 36
   <211> 1191
   <212> DNA
   <213> Artificial Sequence
<220>
   <223> Description of Artificial Sequence:IG6 Fusion
   Protein coding sequence
<400> 36
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgcag 120
   gtggggcagg tggagctggg cgggggccct ggtgcaggca gcctgcagcc cttggccctg 180
   gaggggtccc tgcagaagcg tggcactaac atgttcacct atgaaattgc tccagtattt 240
   gtgcttttgg aatatgtcac actaaagaaa atgagagaaa tcattggctg gccagggggc 300
   tctggcgatg gaggcggtat gaacattctg ctgcagtatg ttgttaaaag cttcgataac 360
   atgtatgcca tgatgatcgc gcgctttaaa atgttcccgg aagttaaaga aaaaggtatg 420
   gccgcgctgc cgcgtctggg aggcggtatt gcctttacct ctgaacatag ccatttttct 480
   ctgaaaaaag gagctgcagc cttagggatt ggaacagaca gcgtgattgg aggcggtatt 540
   gagcatgacc ctcggatgcc agcctacata gccacgcagg gcccgctgtc ccataccatc 600
   gcagacttct ggcagatggt gtgggagagc ggctgcaccg tcatcgtcat gctgaccccg 660
   ctggtggagg atggtgtcaa gcagtgtgac cgctactggc cagatgaggg tgcctccctc 720
   taccacgtat atgaggtgaa cctggtgtcg gagcacatct ggtgcgagga ctttctggtg 780
   cggagcttct acctgaagaa cgtgcagacc caggagacgc gcacgctcac gcagttccac 840
   ttcctcagct ggccggcaga gggcacaccg gcctccacgc ggcccctgct ggacttccgc 900
   aggaaggtga acaagtgcta ccggggccgc tcctgcccca tcatcgtgca ctgcagtgat 960
   ggtgcgggga ggaccggcac ctacatcctc atcgacatgg tcctgaaccg catggcaaaa 1020
   ggagtgaagg agattgacat cgctgccacc ctggagcatg tccgtgacca gcggcctggc 1080
   cttgtccgct ctaaggacca gtttgaattt gccctgacag ccgtggcgga ggaagtgaat 1140
   gccatcctca aggccctgcc ccagcaccat caccatcacc attaaggatc c          1191
<210> 37
   <211> 1344
   <212> DNA
   <213> Artificial Sequence
<220>
   <223> Description of Artificial Sequence:IG7 Fusion
   Protein coding sequence
<400> 37
   catatgttcg ttaaccagca tctgtgtggc tctcacctgg ttgaagccct gtatctggtt 60
   tgcggtgaac gcggcttttt ctacaccccg aaaacccgtc gtgaagcgga agatctgcag 120
   gtggggcagg tggagctggg cgggggccct ggtgcaggca gcctgcagcc cttggccctg 180
   gaggggtccc tgcagaagcg tggcactaac atgttcacct atgaaattgc tccagtattt 240
   gtgcttttgg aatatgtcac actaaagaaa atgagagaaa tcattggctg gccagggggc 300
   tctggcgatg gaggcggtat gaacattctg ctgcagtatg ttgttaaaag cttcgataac 360
   atgtatgcca tgatgatcgc gcgctttaaa atgttcccgg aagttaaaga aaaaggtatg 420
   gccgcgctgc cgcgtctggg aggcggtatt gcctttacct ctgaacatag ccatttttct 480
   ctgaaaaaag gagctgcagc cttagggatt ggaacagaca gcgtgattgg aggcggttac 540
   attcctccaa gcttgcgtac tctggaagac aatgaagaac gcatgagccg tctgtctaaa 600
   gttgccccgg ttattaaagc gcgcatgatg gaatatggca ccaccatggt tagctaccag 660
   ccgctgggtg ataaagttaa cggaggcggt attgagcatg accctcggat gccagcctac 720
   atagccacgc agggcccgct gtcccatacc atcgcagact tctggcagat ggtgtgggag 780
   agcggctgca ccgtcatcgt catgctgacc ccgctggtgg aggatggtgt caagcagtgt 840
   gaccgctact ggccagatga gggtgcctcc ctctaccacg tatatgaggt gaacctggtg 900
   tcggagcaca tctggtgcga ggactttctg gtgcggagct tctacctgaa gaacgtgcag 960
   acccaggaga cgcgcacgct cacgcagttc cacttcctca gctggccggc agagggcaca 1020
   ccggcctcca cgcggcccct gctggacttc cgcaggaagg tgaacaagtg ctaccggggc 1080
   cgctcctgcc ccatcatcgt gcactgcagt gatggtgcgg ggaggaccgg cacctacatc 1140
   ctcatcgaca tggtcctgaa ccgcatggca aaaggagtga aggagattga catcgctgcc 1200
   accctggagc atgtccgtga ccagcggcct ggccttgtcc gctctaagga ccagtttgaa 1260
   tttgccctga cagccgtggc ggaggaagtg aatgccatcc tcaaggccct gccccagcac 1320
   catcaccatc accattaagg atcc                                        1344

## Description

### BACKGROUND OF THE INVENTION

The discussion in this section is not limited to subject matter that qualifies as "prior art" against the present invention. Therefore, no admission of such prior art status shall be implied or inferred by reason of inclusion of particular subject matter in this discussion, and no declaration against the present inventors' interests shall be implied by reason of such inclusion.

### Diabetes Mellitus

Diabetes mellitus is the most common endocrine disease, and is characterized by abnormalities of glucose metabolism. The abnormal glucose metabolism associated with this disease results in hyperglycemia (high blood glucose levels) and eventually causes complications of multiple organ systems, including eyes, kidneys, nerves, and blood vessels. Patients with persistent hyperglycemia or abnormal glucose tolerance are generally diagnosed with the disease, although most commonly patients initially present with excessive urination (polyuria) and frequent drinking due to extreme thirst (polydipsia). These typical initial symptoms result from the osmotic effects of hyperglycemia.

The pathogenesis of diabetes mellitus is typically associated with pancreatic dysfunction, particularly of the beta cells of the pancreatic islets of Langerhans. This dysfunction may lead to destruction of the islet beta cells, which produce insulin, a glucose regulatory peptide hormone. Diabetes mellitus has been generally categorized as insulin dependent or type 1, versus non-insulin dependent, or type 2. However, this terminology has evolved as the disease has become better understood. For example, it has been found that in some patients suffering from non-insulin dependent diabetes, the disease progresses into an insulin dependent form, while in other patients insulin dependence does not develop.

Patients are thus often categorized in terms of the mechanisms of pathogenesis of islet destruction, and the designation type 1 is now used to refer to autoimmune islet pathogenesis, i.e., to diabetes caused by islet-specific autoimmune attack, and is so used herein. The term insulin dependent diabetes mellitus (IDDM) refers to Type 1 diabetes that has progressed to a stage where enough autoimmune destruction of the pancreatic beta cells has occurred to produce overt symptoms. The term pre-IDDM refers to an autoimmune condition that can be detected by biopsy or by analysis of autoimmune responses, in which pancreatic islet beta cells are being subject to a specific autoimmune attack to an extent where some cells may be subject to destruction. In pre-IDDM, however, the destruction (if any) has not progressed to an extent sufficient to require the administration of insulin. Since there can be a point in the early stages of Type 1 diabetes in which overt symptoms are observed but some islet function remains (known as the "honeymoon period", not all Type 1 diabetes is classified as IDDM, and not all pre-IDDM presents without overt symptoms.

Complications of Type 1 Diabetes The metabolic complications associated with the abnormal metabolism caused by insulin insufficiency can affect numerous organ systems. The most common acute metabolic complication is that of diabetic ketoacidosis, characterized by severe hyperglycemia (and resulting hypovolemia caused by osmotic diuresis) as well as metabolic acidosis induced by excess free fatty acid release and the production of ketone bodies.

In addition to the acute metabolic complication of ketoacidosis, the diabetic patient is susceptible to a series of late complications that cause considerable morbidity and premature mortality. Atherosclerosis occurs more extensively and earlier in diabetics than in the general population as a result of abnormalities in both glucose and lipid metabolism. This vascular pathology can lead to, *inter alia,* coronary artery disease, stroke, and peripheral vascular disease with gangrene. Retinopathy is another vascular complication of diabetes. Diabetic retinopathy is a leading cause of blindness, and is initiated by increased permeability of retinal capillaries which can progress to occlusion, hemorrhage, aneurysm formation, and neovascularization known as proliferative retinopathy.

In addition to vascular complications, kidney and neurological diseases (nephropathies and neuropathies) are common complications of diabetes. Diabetic nephropathy causes about half of end-stage renal disease in the United States. Histologically, the nephropathy is characterized by glomerular basement membrane widening and mesangial thickening. Initial signs include increasing proteinuria, with azotemia ultimately leading to renal failure. Diabetic neuropathy can affect any part of the nervous system, with the possible exception of the brain. The neuropathy is most commonly seen as peripheral polyneuropathy, with symptoms including numbness, paresthesias, severe hyperesthesias, and pain. Autonomic neuropathy can cause gastrointestinal dysfunction, orthostatic hypotension, bladder dysfunction or paralysis, and impotence. Diabetic foot ulcers represent a special problem of diabetics, and appear to be due primarily to abnormal pressure distribution secondary to diabetic neuropathy. The ulcerous lesions are often worsened by concomitant peripheral vascular disease and infection.

As mentioned above, meticulous control of blood glucose has been associated with amelioration of the late complications of Type 1 diabetes, suggesting that that preservation or restoration of beta cell function could reduce or eliminate the majority of the pathologic complications of the disease.

Pathogenesis of Type 1 Diabetes Type 1 diabetes only develops in genetically susceptible individuals, and symptoms generally appear before age 40, with the peak incidence of onset of overt symptomology occurring in the second decade of life. The pathogenesis of Type 1 diabetes is characterized by an initial phase of leukocyte infiltration into the islets, referred to as insulitis, followed over a period of time by the actual destruction of the islet beta cells by autoimmune attack. The insulitis phase is characterized by infiltration of pancreatic islets by both lymphocytes and cells of the monocyte/macrophage lineage, and entails both cell-mediated inflammation as well as attack by islet-specific cytotoxic antibodies. Overt clinical symptoms of diabetes mellitus are generally manifested when over 90% of the islet beta cells are destroyed; however, as discussed more fully below, it is now possible to accurately detect individuals undergoing earlier stages of type 1 pathogenesis, i.e., before enough islet beta cells have been lost to produce overt clinical symptoms.

The autoimmune process is generally thought to be induced by an environmental stimulus. One reason for this belief is that an identical twin has only a fifty / fifty chance of developing IDDM if his identical sibling has the disease.

T Cells The autoimmune destruction of the beta cells of the pancreatic islets in Type 1 diabetes is believed to be initiated by white blood cells (leukocytes), most importantly T cells. T cells, or T-lymphocytes, are mononuclear white blood cells that provide many essential immune functions. The importance of T cells in human autoimmune diseases has been increasingly appreciated in the past two decades. Studies using treatments that result in generalized immunosuppression have defined a critical role for a subset of T cells, known as CD4⁺ or helper T cells, as primary regulators of all immune responses (both cellular and humoral) to protein or peptide antigens.

T cells mediate tissue injury by indirect and direct means. T cells of both CD8⁺ (cytotoxic) and CD4⁺ (helper) subsets secrete a variety of inflammatory cytokines that can damage tissues indirectly by activating various other types of white blood cells. Examples of such T cell effects include activation of antibody secreting B cells (stimulating humoral immune activity) and activation of macrophages, which can cause acute tissue damage and inflammation by releasing hydrolytic enzymes, reactive oxygen species, and additional pro-inflammatory cytokines. In addition to these indirect effects of T cell activity, direct tissue damage can be mediated by CD8⁺ cytotoxic T cells attacking cells displaying target antigens.

One unique aspect of the physiology of T cells is the presence of membrane bound antibody-like binding structures called T cell receptors (TCRs) on their cell surfaces. Like antibodies, TCRs bind with high specificity to particular antigens. Like antibody-producing cells, which develop as multitudinous clones of cells, each clone producing antibodies with unique specificities, T cells develop as a vast number of distinct clones, and any particular T cell clone expresses a single type of TCR with a defined binding specificity. T cell clones with TCRs that bind specifically to self antigens are responsible for the development of autoimmune diseases.

Studies of the interactions of antibodies and TCRs with their specific antigens have shown that a particular polypeptide antigen typically comprises numerous submolecular features, known as epitopes, that each can serve as a distinct binding site for a particular antibody or TCR.

T Cells and Autoimmune Diseases In autoimmune diseases, only a limited number of T cell clones, reactive with various epitopes of a small number of autoantigens, become activated and are involved in pathogenesis. Even in individuals suffering from autoimmune diseases, only a small percentage of T cell clones (0.1-1%) are known to recognize autoantigens.

Various mechanisms have been postulated to play a role in the pathogenic activation of disease-causing autoreactive T cells. Primary activation of antigen presenting cells (APCs) by infection or local inflammation is implicated in one such mechanism. APCs activated in this way can then provide powerful co-stimulation for hitherto unreactive T cells.

Other proposed mechanisms involve the polyclonal activation of previously quiescent autoreactive T cells by superantigens, such as bacterial toxins; or a coincidental molecular mimicry between foreign and self antigens (Abbas et. al. 1994). In this last case, the host immune system mounts a response to an epitope on a protein expressed by a pathogen, such as a virus, that resembles a homologous epitope on a host protein. Autoimmune attack then results from the cross-reactive immune response that ensues.

In addition to external factors, underlying the emergence of all T cell-mediated autoimmune disease is a complex pattern of inherited susceptibility determined by multigenic factors. For further discussions of these various factors, Steinman, 1995, reviews current theories of autoimmunity.

Alterations in the T cell repertoire occur naturally during T cell development. Only a small fraction of thymocytes (immature T cells) survive the intrathymic development and selection events that result in emigration of developing T cells to the peripheral circulation and the completion of their maturation (von Boehmer, 1988; Marrack and Kappler, 1987). Experimental evidence strongly suggests that a large number of thymocytes that bear receptors for autoantigens are initially present in the thymus. Recent studies have yielded evidence suggesting that a process referred to as programmed cell death, or apoptosis, destroys these autoreactive thymocytes in the thymus while sparing thymocytes that are not autoreactive. Apoptosis thus plays a large role in shaping and maintaining the T cell repertoire and contributes to the establishment of self-tolerance by actively eliminating cells expressing autoreactive TCRs.

It has recently been discovered that T cells are sensitive to apoptotic cell death induced by a variety of stimuli at multiple points in their lifespan (see, for example, Lenardo 1991; Boehme and Lenardo 1993; Critchfield et al. 1994). Positive selection factors are also believed to play a role in regulating the survival of specific T cell clones. The reduction or expansion of the number of individual T cells of a particular clone in an organism by these and other mechanisms serve to modulate the responsiveness of the organism's immune system to a particular antigen. It is now firmly established in several autoimmune disease models, as well as in certain viral infections, that apoptosis can be induced (upon exposure to antigen under certain defined conditions) in mature peripheral antigen-specific T lymphocytes as well as in immature thymocytes.

Apoptosis occurs in many biological systems (see, for example, Kerr et al. 1991; Lockshin and Zakeri, 1991; Cohen et al. 1992; Duvall and Wyllie, 1986; Cotter et al. 1990). A cell undergoing apoptosis undergoes a specific program of events -- cellular and biochemical processes that depend upon active metabolism and contribute to the cell's self-destruction. In apoptotic T cells, the nucleus shrinks, the chromatin condenses, the genetic material (DNA) progressively degrades into small (nucleosomal repeat sized) fragments, there is cytoplasmic compaction, the cell membrane forms blebs, and the cell eventually collapses (Kawabe and Ochi, 1991; Smith et al. 1989). Cells cannot recover from apoptosis, it results in irreversible cell death (Kawabe and Ochi, 1991; Smith et al. 1989).

Recent reports have suggested a role for the TNF-related cytokine known as the FAS ligand and its receptor, CD95 (the FAS receptor), in the induction of apoptosis in T cells (Crispe et al. 1994; Nagata and Suda, 1995; Strasser, 1995; Dhein et al., 1995; Brunner et al., 1995; and Ju et al., 1995).

Islet Beta Cell Autoantigens As discussed above, the onset of Type 1 diabetes is considered to be mediated by T cells. The disease is believed to be a consequence of inappropriate T cell responses specific to certain islet beta cell proteins that act as autoantigens. In addition to autoreactive T cells, autoantibodies against various self antigens have also been reported in IDDM patients. The antigens reported to be bound by these autoantibodies include many of those that have been reported to be recognized by autoreactive T cells.

Autoantigens that are subject to autoimmune responses in Type 1 patients include the 64-65kDa GAD (glutamate decarboxylase) and the 67kDa GAD autoantigens; insulin; sialyglycolipid; a 38kD antigen from the secretory granules of beta cells; an antigen cross reactive with antibodies to bovine albumin known as the beta cell p69 protein, PM-1, or disease-modifying antigen, a beta cell cytoskeletal protein known as peripherin, glucose transporter proteins, including GLUT-2; heat shock protein 65 (HSP 65), including the p277 peptide; carboxypeptidase H; a 52Kd molecular mimic of Rubella virus antigen; a beta cell membrane associated protein of 150kDa; a protein antigen located at the secretory pole of the rat insulinoma cell line RINm38, referred to as the RIN polar antigen; and (at first) poorly characterized antigens isolated by immunoscreening of an islet cDNA expression library, referred to as ICA12 and ICA512. ICA512, now also known as IA-2, is immunologically related to phogrin, which is also subject to autoimmune responses in Type 1 patients (Hatfield et al., 1997).

The relative importance of these various autoantigens to autoimmune pathogenesis, and the timing with which each plays a role during the course of disease onset and development, are the subject of considerable uncertainty and consequent controversy in the art. Further uncertainty stems from the fact that each supposed autoantigen comprises numerous epitopes, some of which may be have disease promoting effects while others may have disease suppressive effects.

While not wishing to be bound by any particular theory of operation, in accordance with certain aspects of the invention insulin and GAD are believed to provide the most effective therapeutic effects on the development of Type 1 diabetes of any of the autoantigens implicated as playing a role in the pathogenesis of the disease.

Autoantibodies to 64-65kD GAD (hereinafter GAD 65) normally are detected before the onset of clinical insulin dependent Type 1 diabetes mellitus, and among nondiabetic relatives of patients with IDDM as well as others at risk. These autoantibodies have been suggested to be the best predictive autoantibody marker for impending Type 1 diabetes.

GAD 65 and GAD 67 are encoded by different genes on different chromosomes, the genes being about 70% homologous. Human islets only express GAD 65, although both protein forms are found in the brain. Evidence of lymphocyte specific immunity to GAD 65 has been demonstrated and found to be closely associated with IDDM. Recent studies in the NOD mouse model of diabetes have indicated that T cell responses to GAD 65 precede those to other putative autoantigens and that early induction of T cell tolerance to GAD 65 can prevent onset of disease.

Kaufman et al (1993) and Tisch et al (1993) have presented data that suggest that GAD responses are the most important in disease development, as they were reported to arise first during the development of Type 1 diabetes, with responses to other beta cell autoantigens only appearing much later in the course of the disease, with insulin reactivity being amongst the last to appear. These findings were interpreted as indicating that GAD 65 is the key autoantigen in Type 1 diabetes, and that modulation of autoimmune reactivity with GAD would be the most appropriate target for reducing disease pathology. In accordance with this theoretical understanding of disease progression, modulation of insulin reactive T cells would be closing the barn door after the horses had gone, the anti-insulin reactions being observed so late in disease progression that their modulation would not be expected to affect the onset or severity of disease.

Insulin autoantibodies (IAA) can be detected in around 50% of new onset patients, and are highly associated with islet cell autoantibodies (ICA) and the HLA-DR4 phenotype. Other studies suggest that individuals with both ICA and IAA have a much higher risk for developing overt Type 1 diabetes than those with either marker alone. T cell responses to insulin as an autoantigen have also been described. In one study cellular responses to human insulin were present in almost 90% of ICA-positive first degree relatives of IDDM patients. Also, as discussed below in the examples, insulin reactive T cells from diabetic NOD mice can transfer diabetes to non-diabetic NOD mice.

Responses of T cells from Type 1 diabetes patients or from at-risk individuals to undefined islet cell preparations have suggested that T cells also respond to other islet cell antigens. These include a 38kD antigen from the secretory granules of beta cells, and serum albumin. In addition, heat shock protein (HSP) 65 has been implicated as a T cell autoantigen based upon the finding that HSP-specific T cells transfer disease in NOD mice.

Carboxypeptidase H is a molecule found in islet secretory granules and is associated with the production of peptide hormones and neurotransmitters. It was identified as a potential islet autoantigen by the screening of cDNA expression libraries with sera from IDDM or pre-IDDM patients.

Several other putative islet cell antigens, such as ICA12 and ICA512, have also been identified by screening of cDNA expression libraries.

Intra-antigenic and inter-antigenic spread of autoreactivity ("epitope spreading") are related phenomena associated with autoimmune diseases in which additional epitopes within an antigen, or additional antigens within a target tissue, become targeted by autoreactive T cells during disease progression. Such antigen spreading has been observed during the course of the inflammatory autoimmune process in the murine models of experimental allergic encephalomyelitis (EAE) and insulin-dependent diabetes (Lehmann et al. 1992; McCarron et al. 1990; Kaufman et al. 1993; Tisch et al. 1993).

These findings of antigen/epitope spreading suggest that for a therapeutic treatment to provide effective immune tolerance to islet beta cell autoantigens, the treatment will need to target a heterogeneous population of specific autoreactive T cells. Therefore, in order for antigen administration to be maximally effective in the prevention and treatment of Type 1 diabetes, it is desirable that a plurality of the immunodominant epitopes of both insulin and GAD 65 be presented to the disease producing autoreactive T lymphocytes.

Prediction and Diagnosis of Type 1 Disease As discussed above, there is a genetic aspect to the incidence of Type 1 diabetes. Accordingly, genetic tests can identify certain individuals at increased risk of developing the disease (see, for example, Walston et al. 1995). Furthermore, individuals with a known family history of the disease can be monitored for early, preclinical signs of disease development, e.g., by monitoring levels of the autoantibodies and autoreactive T cells discussed herein.

Autoantibodies Among the autoantibodies known to be associated with Type 1 diabetes, those directed against GAD 65 are the ones that appear earliest and are present in the largest number of patients. Overall, recent studies have shown that over 80% of individuals with preclinical diabetes have GAD-specific autoantibodies. In this case an individual with preclinical disease is defined as a first degree relative of a Type 1 diabetes patient with ICA. The antigens identified by ICA are ill-defined, but together with IAA and GAD-specific autoantibodies, they yield a high predictive value for onset of diabetes in preclinical individuals. Interestingly, in actual early onset disease, the frequency of GAD-specific antibodies declines. This could be due to the fact that GAD 65 reactivity declines with beta cell destruction.

Prediction of Type 1 diabetes may also be facilitated by monitoring of the subject's blood sugar levels, preferably, in conjunction with the administration of a glucose tolerance test to the subject. Such procedures are preferably carried out in combination with the monitoring of titers of the subject's circulating IAA, ICA, and GAD autoantibodies.

In accordance with the present invention, the chimeric proteins of the invention are fusion proteins that may be used as antigenic substrates for the detection of circulating autoantibodies, particularly IAA and/or GAD 65 autoantibodies, in diagnostic assays such as Western blot, ELISA, RIA, ELISPOT, and the like.

T cells Assays for the detection of T cells with specific reactivities are well known in the art, and include the mixed lymphocyte reaction (MLR) and the ELISPOT assay. ELISPOT assays are described, for example in Taguchi et al., J Immunol Meth 1990, 128:65 and Sun et al., J Immunol 1991 146:1490. In accordance with the invention, the chimeric fusion proteins of the invention may be used as substrates in such assays for the detection and quantification of insulin reactive T cells and/or GAD 65 reactive T cells and/or IA-2 reactive T cells.

Current Methods for Prevention and Treatment of Type 1 Diabetes. While diabetes has been studied for centuries, only a few effective treatments are available for type 1 disease. The first line of treatment is diet, with appropriate caloric intake based on ideal body weight and a defined distribution among protein, glucose, and fat. However, in IDDM patients, the most important component of therapy is the administration of insulin, the goal of which is to maintain glucose levels as close to the normal range as possible throughout the day. Insulin is available in rapid, intermediate, and long-acting formulations which vary in onset, peak, and duration of action, and can be used in varying schedules of administration to attempt to optimally regulate plasma glucose levels.

Intensive insulin therapy refers to a rigorous regimen of administration of hormonally effective insulin and monitoring of blood sugar levels. This regimen is designed to control blood glucose as precisely as possible. The results of the multicenter Diabetes Control and Complication Trial established that complications of diabetes are significantly diminished by better control of blood glucose levels, and thus demonstrated the desirability of intensive insulin therapy. One problem with this approach is that intensive insulin therapy requires a high level of patient awareness and compliance, as well as a highly skilled care team of physicians, nurses, and dietitians. The goals of intensive insulin therapy are thus extremely difficult to achieve, even with motivated and educated patients. Another problem is that a higher rate of hypoglycemia is seen in such rigorously treated patients than in patients receiving standard, less rigorous, insulin regimens.

The Diabetes Control and Complication Trial highlighted not only the benefit to overall metabolic health of maintaining normal blood glucose levels, but also a fundamental problem associated with the treatment of Type 1 diabetes, namely that the overt symptoms of the disease are manifested only when essentially all of the patients' islets are destroyed. Oral agents for diabetes, such as the sulfonylureas, act primarily by stimulating the release of insulin from dysfunctional beta cells, and thus are not useful for most patients with type 1 disease, i.e. for those patients with IDDM.

A major goal in the treatment of diabetes has been to develop therapies capable of aborting the autoimmune attack on the islet beta cells prior to their complete destruction, thereby preserving enough endogenous function to maintain normal metabolic control.

Induction of tolerance In the NOD (non-obese diabetic) mouse model of diabetes, it has been shown that oral feeding of insulin delayed the onset and reduced the severity of the disease. The mechanism proposed to explain oral tolerance is that oral antigen administration induces populations of antigen-specific Th2 T cells that secrete antiinflammatory cytokines such as IL-4, IL-10, and TGF-beta. These T cells circulate and are activated to secrete cytokines only in the presence of their specific antigen. Thus, insulin-specific Th2 T cells would be activated only in the pancreas where they would produce suppressive cytokines to modulate the autoimmune process. This mechanism does not require, therefore, that the oral antigen actually represent a disease-specific autoantigen, but rather only that it is expressed in a tissue specific fashion.

In contrast, methods designed to produce T cell tolerance (e.g., by anergy or apoptosis) require the identification of the actual disease-specific autoantigens that are targeted by autoimmune attack. Such antigens are then administered to patients in an appropriate tolerizing fashion (which may also induce non-antigen-specific tolerizing effects). Given that Type 1 diabetes is in significant measure a disease mediated by islet-specific autoreactive T cells, therapy of this type should be feasible in principle. Thus, induction of neonatal tolerance to GAD 65, as referred to above, prevented onset of disease in NOD mice. In addition, injection of crude islet extracts intrathymically, where tolerization of developing T cells takes place, has also protected both NOD mice as well as pre-diabetic BB rats from developing clinical disease.

One approach taken to induce insulin tolerance involves the parenteral administration of insulin, in combination with a conventional adjuvant (e.g., Freund's adjuvant). Typically this approach involves the administration of doses of insulin that would not be large enough to be expected to cause insulin shock in the patient. Notably, the insulin moieties of the chimeric fusion proteins of the present invention are hormonally ineffective, and are thus suitable for use in accordance with the methods of PCT application WO 97/09061, filed in the name of Yi Wang.

Apoptosis Apoptosis is a form of programmed cell death that occurs in many biological systems (Kerr et al., 1991; Lockshin and Zakeri, 1991; Cohen et al., 1992; Duvall and Wyllie, 1986; Cotter et al., 1990). As discussed above, an apoptotic cell undergoes a specific program of events that depend upon active metabolism and contribute to its own self-destruction. T cells that do not undergo apoptosis, but which have become activated, will carry out their "effector" functions by causing cytolysis, or by secreting lymphokines that cause B cell responses or other immune effects (Paul, 1989, pp. 3-38). These "effector" functions are the cause of tissue damage in autoimmune and other diseases. A powerful approach to avoiding disease is thus to permanently eliminate by apoptosis only those T cells reactive with autoimmune disease-inciting antigens, while leaving the majority of the T cell repertoire intact. The use of autoantigens to carry out this approach is described in PCT patent publication No. 94/28926, filed in the name of Michael J. Lenardo, and entitled Interleukin-2 Stimulated T Lymphocyte Cell Death for the Treatment of Autoimmune Diseases, Allergic Disorders, and Graft Rejection, and PCT patent publication No. 94/03202, filed in the name of Michael J. Lenardo, Stefen A. Boehme, and Jeffrey Critchfield and entitled Interleukin-4 Stimulated T Lymphocyte Cell Death.

Transplantation Transplantation of healthy pancreata, pancreatic tissue, or isolated pancreatic islets into patients suffering from Type 1 diabetes provides an effective treatment modality. Unfortunately, the duration of the therapeutic benefit of such transplants is currently limited by the same autoimmune phenomena that cause type 1 disease in the first place. Accordingly, treatment of a diabetic patient using the chimeric fusion proteins of the invention in accordance with the methods of the present invention, when carried out prior to, concomitantly with, and/or shortly after such a transplant, will increase the longevity of such transplants and thereby enhance the therapeutic benefit of such transplantation procedures.

The accompanying figures, which are incorporated in and constitute part of the specification, illustrate certain aspects of the invention, and together with the description, serve to explain the principles of the invention. It is to be understood, of course, that both the figures and the description are explanatory only and are not restrictive of the invention.

WO 96/26218 discloses peptides comprising pro-insulin and an epitope from GAD65 and their potential use in the treatment of IDDM and pre-IDDM.

### BRIEF DESCRIPTION OF THE DRAWINGS

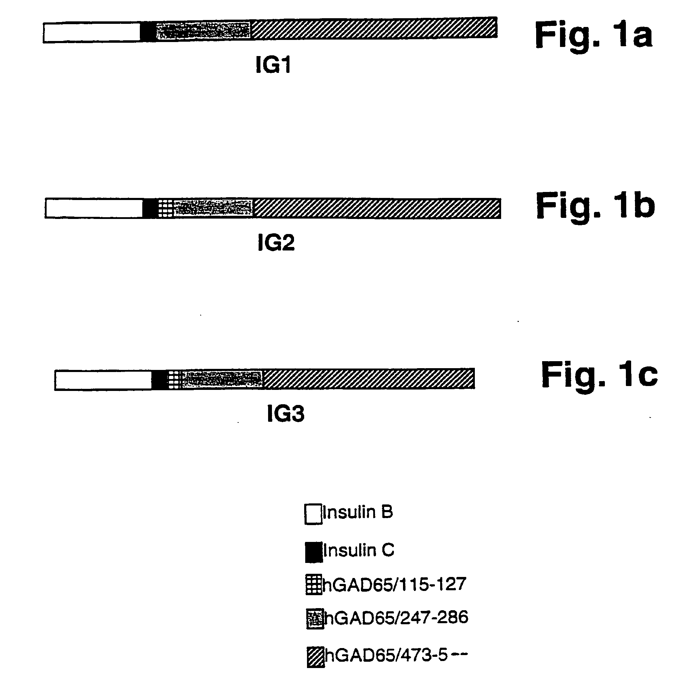

Figure 1a shows a schematic diagram of the IG1 fusion protein (SEQ ID NO:1). The numbers following the backslashes in the legend indicate the GAD 65 peptide moiety to which region corresponds. For hGAD 65/473-5--, 5-- indicates 555.
Figure 1b shows a schematic diagram of the IG2 fusion protein (SEQ ID NO:2). The numbers following the backslashes in the legend indicate the GAD 65 peptide moiety to which region corresponds. For hGAD 65/473-5--, 5-- indicates 555.
Figure 1c shows a schematic diagram of the IG3 fusion protein (SEQ ID NO:3). The numbers following the backslashes in the legend indicate the GAD 65 peptide moiety to which region corresponds. For hGAD 65/473-5--, 5-- indicates 519.

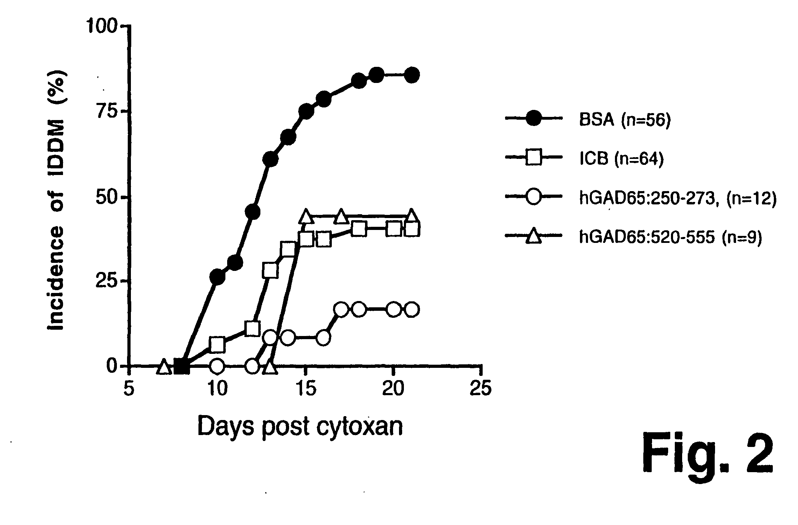

Figure 2 shows the results of CYTOXAN induced IDDM experiments in which NOD mice were treated with bovine serum albumin (BSA, as control) insulin chain B (ICB) human GAD 65 peptide 250-273, or human GAD 65 peptide 520-555 (a peptide with a sequence corresponding to amino acid residues 139-173 of SEQ ID NO:2).

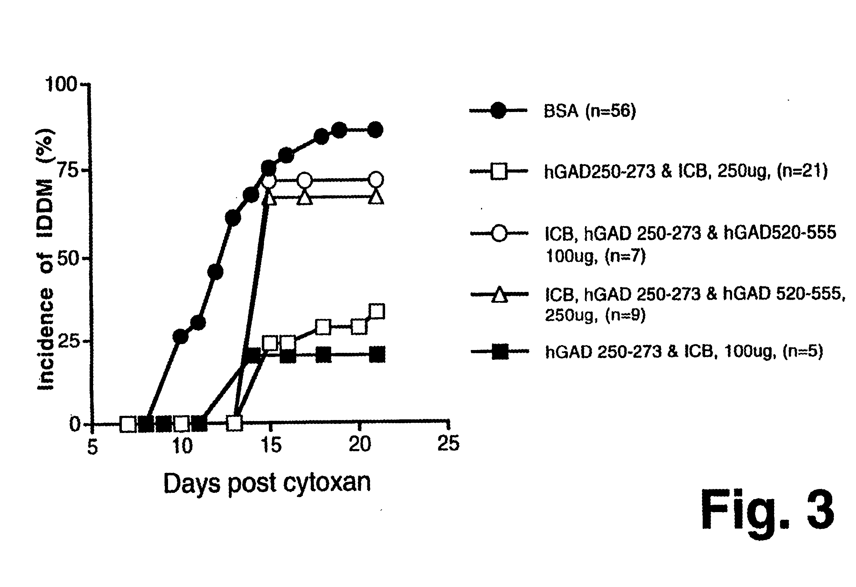

Figure 3 shows the results of CYTOXAN induced IDDM experiments in which NOD mice were treated with bovine serum albumin (BSA, as control) or the following mixtures of insulin chain B (ICB) and human GAD 65 peptides : 1) .100 µg each of ICB and human GAD 65 peptide 250-273, 2) 250 µg each of ICB and human GAD 65 peptide 250-273, 3) 100 µg each of ICB, human GAD 65 peptide 250-273, and human GAD 65 peptide 520-555, and 4) 250 µg each of ICB, human GAD 65 peptide 250-273, and human GAD 65 peptide 520-555.

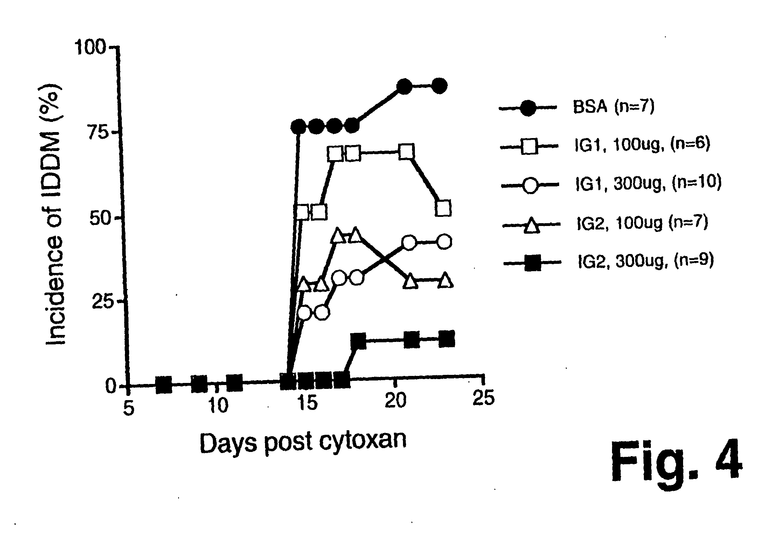

Figure 4 shows the results of CYTOXAN induced IDDM experiments in which NOD mice were treated with bovine serum albumin (BSA, as control) or 100 µg of the IG1 chimeric protein, 250 µg of the IG1 chimeric protein, 100 µg of the IG2 chimeric protein, or 250 µg of the IG2 chimeric protein.

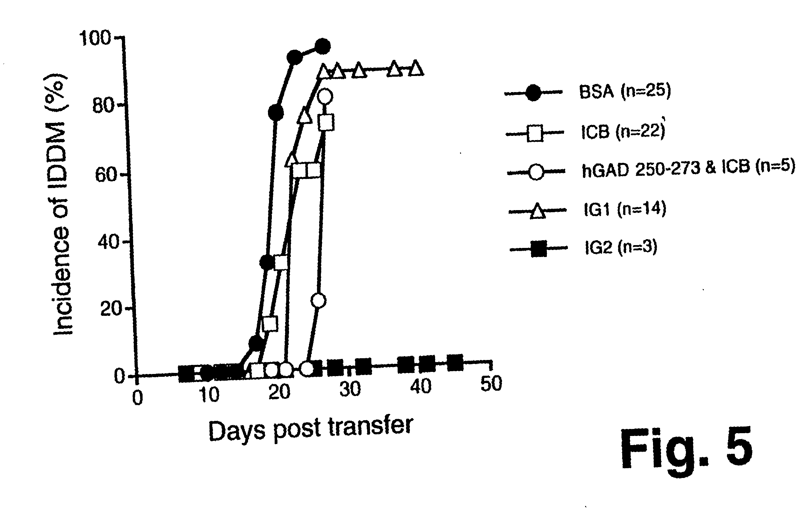

Figure 5 shows the results of the adoptive transfer of IDDM experiments.

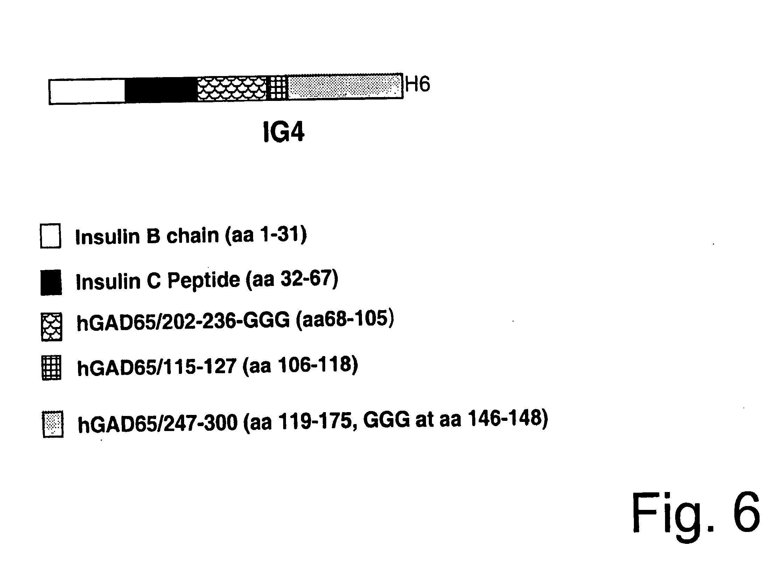

Figure 6 shows a schematic diagram of the IG4 fusion protein (SEQ ID NO:4). The numbers following the backslashes in the legend indicate the position of the indicated peptide moiety component in native human GAD 65 from which its sequence was derived, while the aa numbers in parentheses the corresponding amino acid numbers in SEQ ID NO:4, and the notation GGG indicates the incorporation at that point of a three glycine helix-breaking linker.

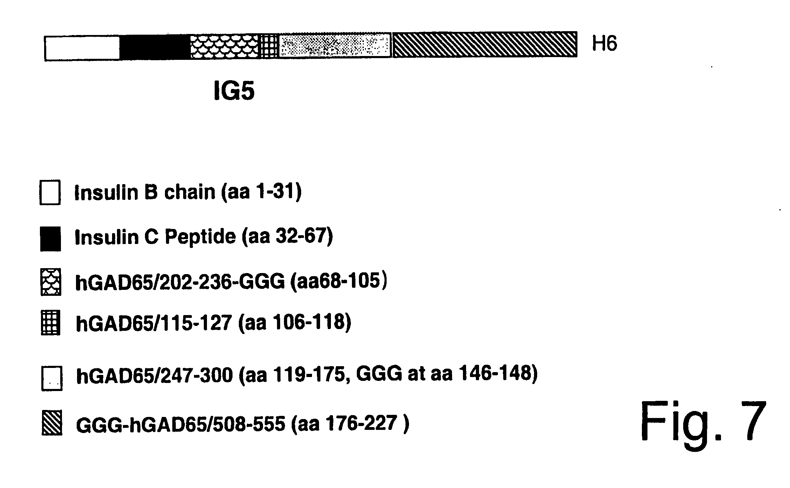

Figure 7 shows a schematic diagram of the IG5 fusion protein (SEQ ID NO:5). The numbers following the backslashes in the legend indicate the position of the indicated peptide moiety component in native human GAD 65 from which its sequence was derived, while the aa numbers in parentheses the corresponding amino acid numbers in SEQ ID NO:5, and the notation GGG indicates the incorporation at that point of a three glycine helix-breaking linker.

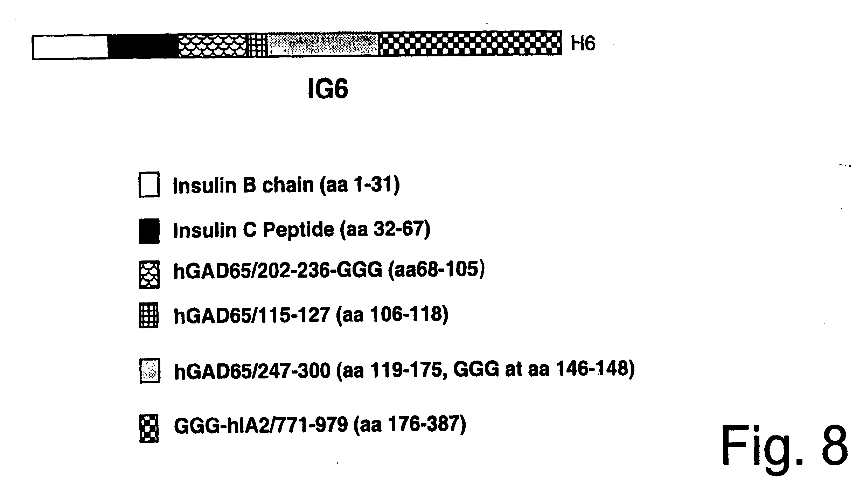

Figure 8 shows a schematic diagram of the IG6 fusion protein (SEQ ID NO:6). The numbers following the backslashes in the legend indicate the position of the peptide component of the native human protein from which its sequence was derived, while the aa numbers in parentheses the corresponding amino acid numbers in SEQ ID NO:6, and the notation GGG indicates the incorporation at that point of a three glycine helix-breaking linker. As indicated in the legend, IG6 comprises, in addition to the indicated portions of human insulin and human GAD 65 (hGAD 65), a C-terminal portion of human IA-2 (hIA2) spanning amino acids 771-979 of the native human protein (amino acids 176-387 of SEQ ID NO:6), with a three glycine helix-breaking linker incorporated at the N-terminus of this portion of IA-2.

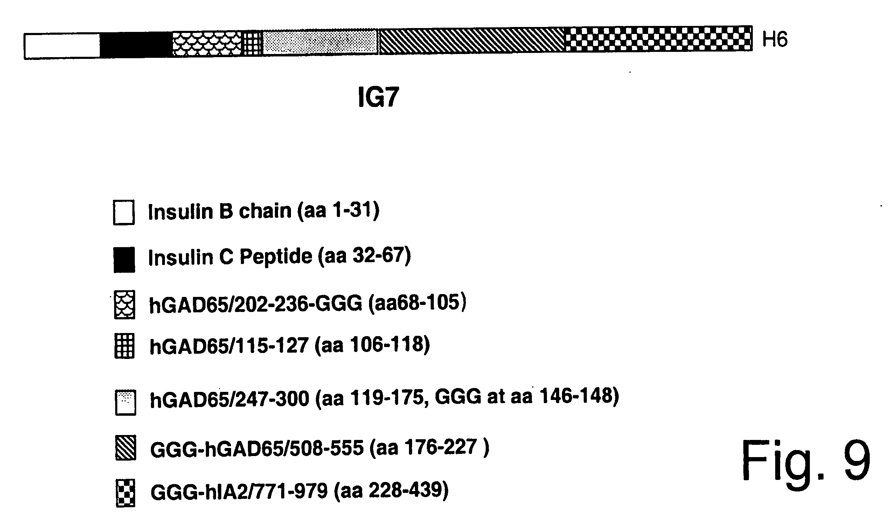

Figure 9 shows a schematic diagram of the IG7 fusion protein (SEQ ID NO:7). The numbers following the backslashes in the legend indicate the position of the peptide component of the native human protein from which its sequence was derived, while the aa numbers in parentheses the corresponding amino acid numbers in SEQ ID NO:7, and the notation GGG indicates the incorporation at that point of a three glycine helix-breaking linker. As indicated in the legend, IG7 comprises, in addition to the indicated portions of human insulin and human GAD 65 (hGAD 65), a C-terminal portion of human IA-2 (hIA2) spanning amino acids 771-979 of the native human protein (amino acids 228-439 of SEQ ID NO:7), with a three glycine helix-breaking linker incorporated at the N-terminus of this portion of IA-2.

**SUMMARY OF THE INVENTION** In view of the foregoing, the objects of this invention include the provision of novel chimeric fusion proteins that act as single molecular entities that 1)facilitate diagnosis and prognostic evaluation of individuals suspected of predispostion for the development of IDDM, as well as those suffering from IDDM and/or Stiff-Man syndrome, and 2) to provide enhanced beneficial effects when administered to animals (including human patients) suffering from or at risk of developing autoimmune (Type 1) diabetes. To these ends the invention provides chimeric fusion proteins comprising epitopes of both GAD (glutamate decarboxylase) 65 and insulin. Preferably the GAD and insulin are human GAD and insulin.

In accordance with the invention, the combination of GAD 65 and insulin peptides in a single fusion protein provides a more convenient diagnostic reagent for the detection and prognostic evaluation of diabetes or stiff man syndrome. Discussion of Stiff-Man syndrome may be found, for example in US patent No. 5,691,448.

In accordance with the invention, (and as set forth in the examples, below) the combination of GAD 65 and insulin chains and/or peptides in a single chimeric fusion protein further provides a compound which can be administered so as to furnish more effective immunomodulatory therapeutic treatment than the combination of the same peptides and/or insulin chains as discrete individual peptide moieties and insulin chains. More specifically, preferred chimeric fusion proteins of the invention, when tested in an assay in a mouse model of IDDM, provide a greater reduction in the frequency of onset of diabetes than a control mixture containing equimolar amounts of each of the various discrete individual peptide moieties and insulin chains comprised by the chimeric fusion protein, each of said individual peptide moieties and insulin chains not being covalently bound to any other of said individual peptide moieties and insulin chains in said mixture, wherein the assay is carried out by the repeated parenteral administration of a number of measured doses, each dose being of a predetermined molar quantity of said chimeric fusion protein in a pharmaceutically effective carrier or of said control mixture in the pharmaceutically effective carrier, the administration being at intervals of not less than twelve hours and not more than 72 hours between each of the doses.

As used herein, the phrase "the same peptides and insulin chains as discrete individual peptide moieties," used in comparison to a particular chimeric fusion protein of the invention, indicates a combination of isolated peptides and insulin chains that are not covalently linked together (e.g., by peptide bonds), wherein linking (via peptide bonds) the peptides and insulin chains together in the appropriate order (e.g., the same relative position in the amino terminal to carboxyl terminal sequence as found in full length GAD 65 and full length insulin chains) would produce the particular chimeric fusion protein of the invention.

While not wishing to be bound by any particular theory of operation, it is believed that complex *in vivo* antigen processing and presentation effects are responsible for the unexpected synergistic effects resulting from combining GAD 65 and insulin peptides and/or polypeptides into a single chimeric fusion protein.

The chimeric fusion proteins of the invention each combine in a single molecular entity the key (immunodominant) autoantigenic epitopes of both insulin and GAD 65. In so doing, they provide single component diagnostic and therapeutic compounds. The chimeric fusion proteins of the invention provide enhanced beneficial effects after administration to animals (including human patients) when compared to the administration of combinations of the individual peptides representing the same immunodominant epitopes as are comprised within the chimeric fusion proteins.

The chimeric fusion proteins of the invention comprise insulin chain B (e.g., amino acids 1-31 of human insulin) and preferably further comprise insulin chain C (the "C fragment", e.g., amino acids 32-38 of human insulin). Amino acid sequences of insulin chains are known. See, for example, US patent No. 4,431,740. for the description of insulin chains and the content of the insulin sequences therein. See also US patent No. 5,008,241.

The chimeric fusion proteins of the invention further comprise at least one GAD peptide.

In accordance with the invention, the at least one GAD peptide is a GAD 65 peptide selected from the group consisting of human GAD 65 peptides 115-127 (a peptide corresponding to amino acid residues 39-50 of SEQ ID NO:2), 247-286 (a peptide corresponding to amino acid residues 51-90 of SEQ ID NO:2), and 473-519 (a peptide corresponding to amino acid residues 92-144 of SEQ ID NO:2. Complete amino acid sequences of GAD 65 polypeptides are known. See, for example, US patent No. 5,691,448.

Results of studies described below suggest that the inclusion of human GAD 65 peptide 520-555 (a peptide with an amino acid sequence corresponding to amino acid residues 139-173 of SEQ ID NO:2) has a deleterious effect upon the immunomodulatory outcome of administration of insulin and GAD proteins or peptides. Accordingly, while within the scope of the invention, chimeric fusion proteins comprising human GAD 65 peptide 520-555 are disfavored. The chimeric fusion proteins of the invention do not include peptide sequences corresponding to those human GAD 65 peptides (particularly those amino terminal GAD 65 peptides) identified as inhibiting GAD solubility in US patent 5,691,448 and do not contain peptide regions of GAD 65 that are associated with the pathogenesis of Stiff-Man syndrome. Thus, in accordance with the teachings of Butler et al., 1993, regarding dominant epitopes of GAD recognized by autoantibodies in Stiff-Man syndrome, the chimeric fusion proteins of the invention do not include peptide regions comprising amino acids 1-95 of GAD 65; additionally, they do not include peptide regions comprising either or both of amino acids 475-484 or 571-585 of GAD 65.

Preferably the GAD peptides are arranged adjacently to each other (i.e., without more than about three intervening amino acids between them) in the same order as they are found in GAD 65, and the insulin chains are arranged adjacently to each other in the same order as they are found in preproinsulin. An arrangement where the insulin chains are amino terminal to the GAD peptides is also preferred in certain embodiments of the invention.

In certain preferred embodiments, at least one (preferably each) of the cysteine residues in the amino acid sequences of the various antigens and antigenic peptides combined to form the chimeric fusion proteins of the invention is replaced with an uncharged amino acid (i.e., an amino acid that is uncharged at a pH of between 6 and 7) having a molecular weight of less than about 150. In another preferred embodiment, none of such cysteine residues are replaced, i.e., there are no substitutions made for any cysteine residues present in any of the various antigens and antigenic peptides combined to form the chimeric fusion protein. When cysteine residues are replaced, the uncharged amino acid is preferably a standard amino acid. Preferably the standard amino acid is alanine or serine. Preferably the replacement of cysteine with another neutral amino acid is an epitope neutral replacement, i.e., it does not result in epitope conversion in any of the known immunodominant epitopes of the chimeric fusion protein, particularly those of GAD or insulin.

Detailed discussions of epitope neutral replacements and epitope conversion can be found in WO 9634622 (US application serial No. 08/431,644, filed May 2, 1995 in the names of Steven H. Nye et al.), for example at pages 34-36 of the specification of that application as filed. Those of skill in the art will readily comprehend the application of the teachings therein to the chimeric fusion proteins of the present invention.

It is a further object to provide immunomodulatory methods for both the prevention and treatment of Type 1 diabetes mellitus and the amelioration of the autoimmune defects underlying this disease. Accordingly, it is an additional object of the invention to provide for the use of the chimeric fusion proteins of the invention in the manufacture of immunomodulatory medicaments.

To achieve these and other objects, the invention provides methods of treating a patient in need of such treatment, e.g., a patient selected from the group of patients consisting of patients at risk of developing type 1 diabetes and patients suffering from type 1 diabetes, so as to delay the onset or reduce the symptoms of diabetes in the patient and/or to ameliorate the autoimmune destruction of pancreatic beta cells in treated patients. Such treatment is particularly advantageous, and is preferably carried out, during the "honeymoon period" early in disease progression, before all of the patient's beta cells have been destroyed by the disease, or later in disease progression, when a patient is a candidate for transplantation of islet cells or tissues containing such cells.

These methods comprise the administration of at least one polypeptide of the invention to the patient. In one preferred embodiment, such administration is carried out on a therapeutic T cell modulatory schedule, i.e., a schedule designed to induce apoptosis, anergy, or other modulation of the autoimmune activity of T cells reactive with at least one epitope of the at least one polypeptide. Particulars of such therapeutic T cell modulatory schedules are discussed below. Other preferred methods of treatment of the invention include administration of at least one polypeptide of the invention to the patient via oral, intravenous, or, preferably, subcutaneous routes, or via parenteral administration with or preferably without an adjuvant such as Freund's incomplete adjuvant, Freund's complete adjuvant, alum adjuvant, or other immunogenic adjuvants now known or subsequently developed.

### DETAILED DESCRIPTION OF THE PREFERRED EMBODIMENTS

Chimeric fusion proteins All polypeptides synthesized in biological systems are initially made with an N-terminal methionine residue. The extreme N-terminal and C-terminal amino acid residues are not considered essential to the functional use of the polypeptides of the invention, which, although not particularly preferred, may be produced without such residues. For example, the polypeptides may be chemically synthesized without N-terminal methionines. Chimeric proteins of the invention thus include IG1 (comprising amino acid residues starting at about residue 2 and ending at about residue 153 of SEQ ID NO:1), IG2 (comprising amino acid residues starting at about residue 2 and ending at about residue 173 of SEQ ID NO:2), Preferably IG1 comprises amino acid residues 1-154 of SEQ ID NO:1, IG2 comprises amino acid residues 1-174 of SEQ ID NO:2.

In certain preferred embodiments these chimeric proteins further can comprise a histidine tag (i.e., a stretch of at least five and preferably six contiguous histidine residues, which facilitates the purification of the chimeric fusion protein by metal chelation chromatography). Preferably the histidine tag is located at the extreme C-terminus of the chimeric fusion protein. Histidine tags are discussed in greater detail in WO 96 34622 (US patent applications serial Nos. 08/431,644 and 08/482,114).

Accordingly, in certain of these preferred embodiments IG1 comprises amino acid residues 1-160 of SEQ ID NO:1 and has a predicted molecular weight of about 18.8 kDa, IG2 comprises amino acid residues 1-180 of SEQ ID NO:2 and has a predicted molecular weight of about 21.2 kDa.

**Secondary Structure Considerations** In designing IG4 (SEQ ID NO:4), IG5 (SEQ ID NO:5), and IG6 (SEQ ID NO:6), particular attention was paid to the predicted secondary structures of the polypeptides of the invention. IG4 was initially designed by hypothetically joining peptide epitopes of interest to form a single hypothetical sequence, IG4NHB (SEQ ID NO:8).

Secondary structure prediction of IG4NHB (SEQ ID NO:8) according to Chou and Fasman, 1978; Chou 1990; and Garnier et al., 1978; was performed using LASERGENE sequence analysis software (DNASTAR, Madison WI). This algorithm predicts secondary structure of proteins from their amino acid sequences. Other sequence analysis software may also be used for this purpose, including other commercially available software such as GCG or MACVECTOR.

The entire sequence from amino acid 77 (Phe 77) to amino acid 134 (Thr 134) of IG4NHB (SEQ ID NO:8) was predicted to have helix forming characteristics. The propensity for actual long helix formation diminishes very rapidly for sequences higher than 20 amino acids, with the longest unbroken helices typically containing no more than 26 amino acids. The 57 amino acid helix-forming sequence from amino acid 77 to 134 of IG4NHB (SEQ ID NO:8) would thus be expected to break at unpredictable, if not random, points so as to form a variety of different structures. Such structures are undesirable in the chimeric fusion proteins, as they would be likely to be prone to uncontrollable aggregation and would be expected to differ from the native secondary structures of the epitopes in the isolated peptides comprised by the chimeric fusion proteins and in the native proteins from which the peptides are derived (e.g., insulin, GAD 65, or IA-2), which native secondary structures are preferred for the epitopes contained within the chimeric fusion proteins. Therefore, in certain preferred embodiments, helix breakers (see following paragraph) are inserted between the epitopes to 1) block the formation of a very long helix and 2) to predictably separate the epitopes into distinct structural entities with distinct secondary structures.

Helix breakers are amino acids, or groups of sequential amino acids that act to block the propagation of helical secondary structures in nascent polypeptide chains. Gly and Pro are known to be strong helix breakers, Asn and Tyr are weak helix breakers. The insertion of any of these amino acids or combinations thereof in a polypeptide will tend to cause a nascent polypeptide secondary structure helix to terminate at the point of the insertion. To assure helix termination and the separation of the desired epitopes, a helix breaker that is at least two amino acids long (wherein each of the amino acids is the same or different from the other and is selected from the group consisting of Gly, Pro, Asn, and Tyr) is preferred, more preferably, such a helix breaker is at least three amino acids long. Most preferably, the helix breaker is exactly three amino acids long. Highly preferred helix breakers are Pro-Pro-Pro (SEQ ID NO:9) and Gly-Gly-Gly (SEQ ID NO:10), with the latter being the most preferred of these.

Dosage In accordance with the present invention, when used as therapeutic agents, the chimeric proteins of the invention are administered to patients in need of such treatment in amounts ranging from about 6.9 pM/kg/patient to about 8.6 µM/kg/patient. Preferably the amounts range from about 34.5 pM/kg/patient to about 5.2 µM/kg/patient. More preferably the amounts range from about 170 pM/kg/patient to about 3.5 µM/kg/patient. Most preferably the amounts range from about 0.5 µM/kg/patient to about 3.5 µM/kg/patient.

In certain of its aspects, the present invention also provides for the repeated administration of doses containing lower amounts of the chimeric fusion proteins of the invention to a patient in need of such treatment. Although less preferred, doses containing amounts of below about 6.9 pM/kg/patient, and as low as about 1 pM/kg/patient may be used in the practice of the present invention.

Preferably the chimeric proteins are administered without the concomitant administration of an adjuvant, however, when used to perform T cell assays (e.g., in transgenic mice such as described in Wong et al. 1998), initial administration to experimental animals is preferably made with adjuvant.

**Administration on Therapeutic T Cell Modulatory Schedules** In accordance with the invention, a therapeutic T cell modulatory schedule involves administration of doses containing the chimeric proteins of the invention, preferably in a pharmaceutically effective carrier, repeatedly to the patient, at least two times at an interval of at least six, and preferably at least twelve hours, with an interval of not more than seven days, preferably not more than 72 hours, more preferably not more than 48 hours, and most preferably not more than 24 hours between doses.

In accordance with the present invention, the chimeric fusion proteins of the invention are preferably administered parenterally without the concomitant administration of an adjuvant. Administration by a parenteral route will typically be via injection such as intravascular injection (e.g., intravenous infusion), subcutaneous injection, or intramuscular injection. Other non-oral routes of administration, e.g., mucosal, inhalation, transdermal ultrasound, and the like, may be used if desired and practicable for the particular polypeptide to be administered.

Although less preferred, the chimeric fusion proteins of the invention may also be administered orally, however dosages for oral administration will typically be one to two orders of magnitude higher that those discussed above under the subheading "Dosage."

Formulations suitable for injection and other routes of administration are well known in the art and may be found, for example, in Remington's Pharmaceutical Sciences, Mack Publishing Company, Philadelphia, PA, 17th ed. (1985). Preferred formulations for parenteral administration of the proteins of the invention are those described for insulin in the USP 23/NF 18 (1995).

Parenteral formulations must be sterile and non-pyrogenic, and generally will include a pharmaceutically effective carrier, such as saline, buffered (e.g., phosphate buffered) saline, Hank's solution, Ringer's solution, dextrose/saline, glucose solutions, and the like. Formulations may also contain pharmaceutically acceptable auxiliary substances as required, such as, tonicity adjusting agents, wetting agents, bactericidal agents, preservatives, stabilizers, and the like.

Further discussions of dosage and administration of polypeptides using therapeutic T cell modulatory schedules may be found in WO 9634622 (US patent applications serial Nos. 08/431,644 and 08/482,114) (discussed above) and WO 9709065 (US patent application serial No. 08/565,769, filed December 1, 1995 in the name of Yi Wang) to more fully describe the state of the art to which the present invention pertains.

Without intending to limit it in any manner, the present invention will be more fully described by the following examples.

**Experimental Methods** Non-obese diabetic (NOD) mice are a strain of mice that are prone to the development of diabetes, and represent an accepted model system for the study of diabetes mellitus. These mice, designated NOD/MrkTacfBR, were purchased from Taconic Farms (Germantown, NY). NOD SCID mice, designated NOD/SCID, are available from the Jackson Laboratories, Bar Harbor, ME. The incidence of diabetes by 200 days of age in these mice is 80% in females and 50% in males. At 110 days of age, fewer than 15% of the male NOD mice became diabetic.

CYTOXAN induction experiments: CYTOXAN (Cyclophosphamide) treatment can increase the incidence and accelerate the onset of diabetes in non-diabetic NOD mice. Randomly selected, non-diabetic male NOD mice, which were 100 to 110 days of age, were injected (ip) with cyclophosphamide (250mg/kg) on day 0.

All the mice were subjected to urine glucose measurement 2-3 times per week for a period of 21 days after cyclophosphamide injection. The onset of diabetes was recorded as the first point at which two consecutive days of positive urine glucose results were obtained. Blood glucose was also measured to confirm the urine glucose results. (ExacTech, MediSense Inc., Cambridge, MA) In all the mice tested with positive urine glucose, blood glucose levels were greater than 150mg/dl. At day 21, these mice were sacrificed and examined histologically.

Experiments in the NOD/SCID Adoptive Transfer Model: Spleen mononuclear cells were harvested from diabetic NOD mice of recent disease onset (diabetic not more than 3 weeks) and about 35 x 10⁶ of these spleen cells in 0.2 ml of PBS were injected intravenously into 6-8 week old NOD/SCID mice. Onset of diabetes was monitored biweekly by urine glucose testing and confirmed by blood glucose testing on day 28 relative to the time of spleen cell transfer. At day 28, these mice were sacrificed and examined histologically.

**Reagents**: CYTOXAN (cyclophosphamide, Mead Johnson oncology products) was dissolved in distilled water (25mg/ml). Oxidized bovine insulin Chain B was purchased from Sigma (catalog no. I-6383). Both insulin chain B and the BSA control protein (Miles Inc., #81-001) were dissolved in PBS/1M HCL, pH2, before dialysis against PBS, pH7.2, sterile filtration, and storage of frozen aliquots.

### EXAMPLES

### Genes for expression of chimeric proteins of the invention

IG1. A synthetic chimeric gene encoding chimeric protein IG1 (SEQ ID NO:1) was constructed in two rounds of overlapping PCR (Ho *et al.,* 1989). Each gene subdomain was synthesized in a standard PCR 100*µ*l reaction using 10 pmole of each of the appropriate oligonucleotide primers, as described below. The 5' gene segment was amplified using the overlapping primers:prIG1 (SEQ ID NO:11), and prIG2 (SEQ ID NO:12). The 3' gene segment was amplified using the overlapping primers prIG3 (SEQ ID NO:13) and prIG4 (SEQ ID NO:14). The prIG1 oligo-nucleotide (SEQ ID NO:11) was designed to allow creation of a unique *Nde*I restriction site at the 5' end. The prIG4 primer (SEQ ID NO:14) included a unique *Bam*HI site, stop codon (TAA) and 18 nucleotides that encoded a histidine tag sequence for purification of the recombinant chimeric protein by metal affinity chromatography. The two subdomains were combined using flanking oligonucleotides prIG5 (SEQ ID NO:15) and prIG6 (SEQ ID NO:16) in a second PCR reaction to yield a 492 bp gene product. The PCR product was cloned into expression plasmid vector pCR2.1 as described by the supplier (Invitrogen, San Diego, CA). Kanamycin-resistant DH10B transformants were selected and the correct clones and orientations determined by restriction and sequence analysis. Restriction fragments from two clones (#6 and #18) were combined to remove undesired mutations. Following nucleotide sequence analysis, an independent plasmid pCR2.1-IG1 was digested with NdeI and BamHI and the 492 bp fragment inserted into compatible sites of the MP4 expression plasmid pET22b-MP4 (Elliott *et al*., 1996), yielding plasmid pIG1. The insert from pCR2.1-IG1 was also subcloned into plasmid vector pBLUSCRIPTSK+ (STRATAGENE CLONING SYSTEMS, La Jolla; CA) to yield plasmid pSK+IG1. The synthetic IG1 gene (SEQ ID NO:17) encodes a chimeric fusion protein with a predicted mass of about 18.8 kDa.

IG2. A synthetic chimeric gene encoding chimeric protein IG2 (SEQ ID NO:2) was constructed by PCR amplification of an internal IG1 gene fragment using pIG1 as a template along with the sense primer prIG7 (5'-AGATCTGATG AACATTCTGC TGCAGTATGT TGTTAAAAGC TTCGATAACA TGTATGCCAT GATG-3' - SEQ ID NO:18; *Bgl*II site underlined) in combination with the anti-sense oligonucleotide primer prIG8 (5'-TGTACAGATA TTCCGCCAGT TCCAGACATT TTTTCAGAGA AAAATGGCTA TGTTCAGAGG TAAAGGCAAT CAGACGCG-3' - SEQ ID NO:19; *Bsr*G1 site underlined). The 197 bp BglII-BsrG1 PCR fragment was subcloned into pCR2.1. Sequence analysis revealed a C>G substitution at position 183 in the coding strand of the 197 bp BglII-BsrG1 PCR fragment for all subclones analyzed, and the plasmid was named pCR2.1-IG7/8C183G. Subsequent analysis revealed an error in the original prIG8 primer sequence (SEQ ID NO:19). A repair primer, prIG12 (SEQ ID NO:20), was synthesized to correct the C>G substitution in pCR2.1-IG7/8C183G. The internal 197 bp BglII-BsrG1 DNA segment was corrected by PCR using pCR2.1-IG7/8C183G as a template along with primers prIG7 (SEQ ID NO:18) and prIG12 (SEQ ID NO:20). The PCR fragments were subcloned into pCR2.1, their sequence was determined using standard dideoxy sequencing to confirm that the desired sequence had been obtained, and a single clone designated pCR2.1-IG7/12 was isolated and amplified.

The 137 bp BglII-BsrG1 restriction fragment in pSK+IG1 was exchanged with the 197 bp BglII-BsrG1 fragment from pCR2.1-IG7/12 to create pSK+/IG7/12. The 552 bp NdeI- BamHI fragment from pSK+/IG7/12 was subcloned into the compatible sites of pIG1 to create plasmid pIG2. The synthetic IG2 gene (SEQ ID NO:21) encodes a chimeric fusion protein with a predicted mass of about 21.2 kDa.

IG3. A synthetic chimeric gene encoding chimeric protein IG3 (SEQ ID NO:3) was constructed by removing a 552 bp *Nde*I-*Bam*HI restriction fragment from plasmid pET22b-IG2 and substituting an *Nde*I-*Bam*HI digested 444 base pair PCR product therefor. This 444 base pair PCR product was made using primers prIG5 (SEQ ID NO:15) and prIG13 (SEQ ID NO:22) with the synthetic IG2 gene as a template. The synthetic IG3 gene (SEQ ID NO:23) encodes a chimeric fusion protein with a predicted mass of about 17.1 kDa.

IG4. A gene sequence encoding chimeric protein IG4 (SEQ ID NO:4) is set forth below as SEQ ID NO:24, and encodes a chimeric fusion protein with a predicted mass of about 19.8 kDa.

IG5. A synthetic chimeric gene encoding chimeric protein IG5 (SEQ ID NO:5) was constructed via ligation of PCR products using primers prIG14 (SEQ ID NO:25), prIG15 (SEQ ID NO:26), prIG16 (SEQ ID NO:27), prIG17 (SEQ ID NO:28), prIG18 (SEQ ID NO:29), prIG19 (SEQ ID NO:30), prIG20 (SEQ ID NO:31), prIG21 (SEQ ID NO:32), prIG22 (SEQ ID NO:33), and prIG23 (SEQ ID NO:34). The synthetic IG5 gene (SEQ ID NO:35) encodes a chimeric fusion protein with a predicted mass of about 25.3 kDa.

IG6. A gene sequence encoding chimeric protein IG6 (SEQ ID NO:6) is set forth below as SEQ ID NO:36, and encodes a chimeric fusion protein with a predicted mass of about 43.7 kDa.

IG7. A gene sequence encoding chimeric protein IG7 (SEQ ID NO:7) is set forth below as SEQ ID NO:37, and encodes a chimeric fusion protein with a predicted mass of about 49.2 kDa.

Expression and purification of recombinant IG fusion proteins. For each IG fusion protein expression plasmid construction that was bacterially expressed, electrocompetent *E. coli* strain BL21(DE3) was transformed with the expression plasmid and ampicillin-resistant colonies were selected for liquid culture. The 1DE3 lysogen in strain BL21(DE3) contains the gene for T7 polymerase behind the *E*. *coli* lacUV5 promoter for efficient expression of target genes under control of the strong bacteriophage T7 transcriptional and translation signals (Studier *et al*., 1990).

The recombinant chimeric fusion protein was purified from solubilized whole cell pellets by immobilized metal affinity chromatography and analyzed by SDS-PAGE/Coomassie blue staining. Four liters cultures were grown to OD₆₀₀ of 0.8 in Terrific Broth (TB) medium (Sambrook et al., 1992). Protein expression was induced for 5 hours with 1mM isopropylthiogalactoside (IPTG). Induced cells were harvested by centrifugation and frozen overnight at -20°C. Cell pellets were thawed at room temperature and homogenized in 10 ml/g of Buffer A (6 M guanidine-HCl/10% glycerol/20 mM Tris-Cl pH 7.8/500 mM NaCl/200 mg sodium sulfite/280 mg sodium tetrathionate) using a TEKMAR homogenizer (The Tekmar Co., Cincinnati, OH). Cells were frozen at -70°C for 1 hour and thawed at room temperature to promote cell lysis. The cell suspension was gently mixed for 30 min at room temperature using a magnetic stirrer. The soluble fraction containing IG protein was collected as the supernatant following centrifugation of the cell lysate at 10,000xg for 30 min at 4°C in a Beckman JA-10 rotor. The supernatant was loaded on a Ni-NTA (QIAGEN Inc., Chadsworth, CA) column previously equilibrated in Buffer A at a flow rate of 8 ml/min. The column was washed to baseline absorbance using Buffer A. The column was further washed with Buffer B (6 M urea/10% glycerol/20 mM Tris-Cl/500 mM NaCl pH 7.8) and contaminating *E*. *coli* proteins were removed with Buffer C (6 M urea/10% glycerol/20 mM Tris-Cl/500 mM NaCl pH 5.0). IG protein was eluted with Buffer D (6 M urea/10% glycerol/20 mM Tris-Cl/500 mM NaCl pH 4.0). All fractions were collected in batch and analyzed on a 4-20% SDS-polyacrlyamide gradient gel in the presence of reductant. The IG containing fractions (Buffer D wash) were concentrated 10-fold using an AMICON STIRCELL using a PM10 membrane. The sample was dialyzed against two changes of MILLI-Q water at 4°C. The IG preparations were filter sterilized and the concentration determined spectrophotometrically using a conversion factor of 1.06 mg/ml/OD₂₈₀. Five micrograms were analyzed under reducing and nonreducing conditions by SDS-PAGE so as to obtain an initial indication of purify and integrity of the protein preparations.

Chimeric fusion protein treatment -- CYTOXAN model: Groups of randomly selected NOD mice were injected intravenously twice daily with either BSA as a control, or GAD peptides, insulin chain B (ICB) or various combinations of GAD peptides and/or insulin chain B at the doses indicated in the figures on days 1, 3, and 5 following CYTOXAN treatment (day 0). Animals that received injections of BSA (controls) manifested a greater than 80% incidence of diabetes by 21 days following CYTOXAN induction (Fig. 2). In contrast, animals treated with either ICB or either of GAD 65 250-273 or 520-555 peptides, experienced reductions to less than 50% incidence of diabetes. The therapeutic effects of treatment with combinations of ICB and the two GAD peptides were then examined (Fig. 3). Reduction in diabetes incidence to 25% or less was achieved by the combination of GAD 250-273 and ICB at doses of 100 ug or 250 ug per injection.

Surprisingly, the addition of GAD peptide 520-555 (amino acid residues 139-173 of SEQ ID NO:2) appeared to inhibit the therapeutic efficacy of the ICB with GAD 65 250-273 combination. Evaluation in the CYTOXAN model of the IG1 and IG2 chimeric fusion proteins of the invention, showed that treatment with either of these polypeptides mediated a dose-dependent reduction in the incidence of diabetes compared to the BSA treated control animals (Fig. 4). In this experiment, 300 ug doses were more effective than 100 ug doses, and IG2 reduced the incidence of diabetes to a greater degree than IG1. Treatment with 300 ug doses of IG2 reduced disease incidence to less than 12%, compared to greater than 80% disease incidence in the BSA treated control animals.

### Chimeric fusion protein treatment -- adoptive transfer model:

Diabetogenic splenic mononuclear cells were harvested from newly diabetic NOD mice (onset less than 3 weeks previous). To initiate the destruction of islet beta cells and the development of diabetes, 8-12 week old NOD/SCID mice were injected intravenously with 35 x 10⁶ diabetogenic spleen cells. The incidence and onset of diabetes were monitored biweekly by urine glucose testing and confirmed by blood glucose testing at the end of the experiment. The average onset time of diabetes after disease induction was approximately 25 days. IV treatment with IG2, IG3, or islet beta cell antigens was initiated on day 3. Animals were treated with 300ugs of antigen twice daily every other day for a period of six days (from day 3 to day 9, where the time of spleen cell transfer was day 0).

The results of these experiments are set forth in Figure 5. They surprisingly demonstrate that only the IG2 chimeric fusion protein prevented the onset of disease in the NOD/SCID recipients. In contrast, in this model only a slight delay in disease onset was observed in recipients of IG1, insulin chain B (ICB), or the combination of ICB and GAD peptide 250-273. These profound differences in the effects of treatment with IG2 as compared to the other treatment regimens was unexpected. Without wishing to be bound by any particular theory of operation, it is believed that this unexpected finding is a result of the in vivo processing of IG2 into unique antigenic peptides that are particularly effective at eliciting a state of immune tolerance protective against autoimmune diabetes, even in the demanding conditions resulting from challenge with heterogeneous T cell populations derived from the spleens of fully diabetic NOD mice.

### REFERENCES

Abbas et. al. 1991. Cellular and Molecular Immunology W.B.Saunders Company, Philadelphia.
Ammerer, 1983. Meth Enzymol 101:192 *et seq*.
Atkinson and Maclaren, 1993. J Clin Invest 92:1608-1616.
Atkinson et al., 1990. Lancet 335:1357-1360.
Atkinson et al., 1990. Diabetes, 39:933-937.
Atkinson et al., 1992. Lancet 339:458-459.
Atkinson et al., 1993. J Clin Invest 91:350-356.
Ausubel et al., 1994. Current Protocols in Molecular Biology, John Wiley & Sons, New York.
Baekkeskov et al., 1982. Nature 298:167-169.
Baekkeskov et al., 1987. J Clin Invest 79:926-934.
Baekkeskov et al., 1990. Nature 347:151-156.
Bock et al., 1992. Lancet 339:1504-1506.
Boehme and Lenardo 1993. Eur J Immunol 23:1552-1560.
Bonifacio et al., 1990. Lancet 335:147-149.
Bowman et al., 1994. Immunol Today 15(3):115-120.
Brunner et al., 1995. Nature 373:441-444.
Butler et al., 1993. J Exp Med 178:2097-2106.
Chang, et al., 1978. Nature 275:615 *et seq*.
Chen et al., 1994. Science 265:1237-1240.
Chou, 1990. Prediction of protein structure and the principles of protein Conformation, Plenum Press 549-586
Chou and Fasman 1978. Adv. Enzymol. 47:45-147.
Cohen et al., 1992. Ann Rev Immunol 10:267 *et seq.*
Coligan et al., 1995. Current Protocols in Immunology John Wiley & Sons, New York.
Conrad et al., 1994. Nature 371:351-355.
Cotter et al., 1990. Anticancer Research 10:1153 *et seq*.
Crispe 1994. Immunity 1:347-349.
Danielet al,. 1996. Proc. Natl. Acad. Sci. USA 93:956-960.
Davis et al. Basic Methods in Molecular Biology, 2nd ed. Appleton and Lange, Norwalk, CT.
De Aizpurua et al., 1992. Proc Natl Acad Sci, USA 89:9841-9845. Dhein et al., 1995. Nature 373:438-441.
Duvall and Wyllie, 1986. Immunol Today 7:115 *et seq.*
Elliott et al., 1996. J Clin Invest 98:1-11.
Evans and Scarpulla, 1989. Gene 84:135 *et seq*.
Farrell, Jr., 1993. RNA Methodologies: A Laboratory Guide For Isolation And Characterization. Academic Press Inc., San Diego, CA.
Foster 1994. in Harrison's Principles of Int Med, Thirteenth Ed., McGraw-Hill, New York, pp. 1979-2000.
Garnier et al., 1978. J. Mol. Biol 120:97-120
Goeddel, et al., 1980. Nucl Acids Res 8:4057 et *seq.*
Griffin and Griffin, Eds., 1994. PCR Technology, Current Innovations. CRC Press, Boca Raton, FL.
Griffin et al, 1995. Am. J. Pathol 147:845-857.
Grosjean and Fiers, 1982. Gene 18:199 et *seq*.
Hanninen et al., 1992. J Clin Invest 90:1901-1910.
Harrison, 1992. Immunol Today 13:348-352.
Harwood, Ed., 1994. Protocols For Gene Analysis: Methods In Molecular Biology, Vol. 31. The Humana Press, Totowa, NJ.
Hatfield et al., 1997. Diabetologia 40:1327-1333
Hernan et al. 1992 Biochemistry 31:8619 *et seq*.
Herold et al., 1992. J Exp Med 176:1107-1114.
Ho et al. 1989. Gene 77:51-59.
Honeyman et al., 1993. J Exp Med 177:535-540.
Huang and Gorman, 1990. Mol Cell Biol 10:1805 *et seq.*
Ju et al., 1995. Nature 373:444-448.
Karjalainen et al., 1992. New Eng J Med 327:302-307.
Karounos and Thomas, 1990. Nature 39:1085-1090.
Kaufman et al., 1992. J Clin Invest 89:283-292.
Kaufman et al., 1993. Nature 366:69-72.
Kaufman et al., 1993. Nature. 366:69-71.
Kawabe and Ochi, 1991. Nature 349:245-248.
Kawasaki et al., 1997. J. Clin. Endocrinol. Metab. 82:375-380.
Kerr et al., 1991. Apoptosis: The Molecular Basis Of Cell Death, Tomei and Cope (eds.), Cold Spring Harbor Laboratory Press, Plainview, New York, pp. 5 *et seq.*
Kim et al., 1993. Immunol Invest 22 (3) :219-227.
Klaus, ed., 1987. Lymphocytes: A Practical Approach. IRL Press, Oxford, England.
Lenardo, 1991. Nature 353:858-860.
Lockshin and Zakeri, 1991. Apoptosis: The Molecular Basis Of Cell Death, Tomei and Cope (eds.), Cold Spring Harbor Laboratory Press, Plainview, New York, pp. 47 *et seq.*
Lohman et al., 1994. Lancet. 343:1607-1608.
Lohman et al., 1996. J of Autoimmunity. 9:385-389.
Lohmann et al., 1996. Hormone & Metabolic Res. 28:357-360.
Luckow, et al., 1988. Bio/Technology 6:47 *et seq*.
Maclaren, et al., PCT patent appl. Int. Pub. No. WO 94/23737.
MacLaren, N and K. Lafferty. 1993. Diabetes. 42:1099-1104.
Maniatis, 1982. Molecular Cloning: A Laboratory Manual. Cold Spring Harbor Laboratory, Cold Spring Harbor, NY.
Marrack and Kappler, 1987. Science 238:1073 *et seq*.
Moir, et al., 1991. Meth Enzymol 194:491-507.
Morgenstern and Land, 1990. Nucl Acids Res 18:3587 *et seq*.
Mueller et al., U.S. Appl. Ser. No. 08/482,114, filed 6/7/95.
Muir, et al., 1995. J Clin Invest 95, pp.628-634.
Mullis et al., Eds., 1994. The Polymerase Chain Reaction Springer-Verlag, New York, NY.
Nagata and Suda, 1995. Immunol Today 16:39 *et seq*.
Naquet et al., 1988..J. Immunol. 140:2569-2578.
Nossal et al., 1992. Diabetologia, pp. 549-559.
Ormerod, Ed., 1994. Flow cytometry: A Practical Approach, 2nd ed.. IRL Press at Oxford University Press, Oxford, England.
Paul, 1989. Fundamental Immunology, 2nd Ed. Paul (ed.), Raven Press, New York.
Quinn, A and E.E. Sercarz. 1996. J. Autoimmunity 9:365-370.
Ramiya et al., 1996. J. Autoimmunity 9:349-356.
Remington's Pharmaceutical Sciences, Mack Publishing Co., Philadelphia, PA, 17th ed. (1985)
Richter et al., 1992. Proc Natl Acad Sci. USA 89:8467-8471.
Rudy et al., 1995. Mol. Medicine 1:625-633.
Russell et. al. 1993 Proc Natl Acad Sci USA 90:4409-4413.
Sambrook et al. 1990. Molecular Cloning: A Laboratory Manual Cold Spring Harbor Press, Cold Spring Harbor, NY.
Sato et al. 1994. J Biol Chem 269:17267 *et seq*.
Schena, et al., 1991. Meth Enzymol 194:389-398.
Schwartz, 1993. Schwartz, RS, "Autoimmunity and Autoimmune Diseases" in Paul, Fundamental Immunology, 3rd Ed. Raven Press, New York, 1993, pp. 1033-1097.
Sercarz et. al. 1959. Nature 184:1080-1082.
Singer et. al. 1994. Immunity 1:365-371.
Smith et al., 1989. Nature 337:181-184.
Solimena and De Camilli, 1993. Nature 366:15-17.
Steinman, 1995. Cell 80:7-10.
Strasser, 1995. Nature. 373:385-386.
Studier et al. 1990. Meth Enzymol 185:60-89.
Sun et al. 1991. Eur J Immunol 21:1461-1468.
Taguchi et al., 1990. J Immunol Meth 128:65-73.
Talib, et al., 1991. Gene 98:289-293.
Tisch et al, 1993. Nature. 366:72-75.
Tisch et al., 1993. Nature. 366:72-75.
USP 23 / NF 18, 1995 The United States Pharmacopeia / The National Formulary; United States Pharmacopeial Convention, Inc., Rockville, MD.
von Boehmer, 1988. Ann Rev Immunol 6:309 *et seq.*
Walston, et al., 1995. N. Eng J Med 333 :343-347.
Walter et al., 1994. J. Clin. Invest. 8:163-166.
Weiner et al., 1997. US Patent No. 5,643,868.
Weir, 1978. Handbook of Experimental Immunology, 3rd ed. Volume 2, Cellular immunology, Blackwell Scientific Publications, Oxford, England.
Wicker et al., 1996. J. Clin. Invest. 98:2597-2603.
Williams et al. 1988. Nucl Acids Res 16:10453 *et seq*.
Wong et al., 1998. J. Clin Invest 102:947-957.
Xie et al., 1997. J. Immunol. 159:3662-3667.
Zhang et al., 1997. Diabetes 46:40-43.
Zhang et al., 1991. Proc Natl Acad Sci, USA 88:10252-10256.

**TABLE 1**

| Table-1: dose dependency of cyclophosphamide induced diabetes in non-diabetic male NOD recipients | | | |
|---|---|---|---|
| CYTOXAN | # Diabetic / Total | % | Mean onset time |
| 200mg/kg | 2 / 7 | 28.6% | 12 |
| 250mg/kg | 5 / 7 | 71.4% | 11 |
| 300mg/kg | 5 / 7 | 71.4% | 12.6 |

## Claims

1. A chimeric fusion protein comprising an amino terminus and a carboxyl terminus, wherein the protein comprises insulin chain B and individual peptide moieties consisting of at least one GAD 65 peptide(s) capable of eliciting a human T-cell response, wherein the insulin chain B and the at least one human GAD 65 peptide(s) are covalently linked and the chimeric fusion protein is capable of eliciting a human T-cell response to insulin chain B and to each of the at least one GAD 65 peptides wherein the chimeric fusion protein is designated IG1 comprising amino acid residues 2 to 154 of SEQ ID NO: 1.

2. A chimeric fusion protein comprising an amino terminus and a carboxyl terminus, wherein the protein comprises insulin chain B and individual peptide moieties consisting of at least one GAD 65 peptide(s) capable of eliciting a human T-cell response, wherein the insulin chain B and the at least one human GAD 65 peptide(s) are covalently linked and the chimeric fusion protein is capable of eliciting a human T-cell response to insulin chain B and to each of the at least one GAD 65 peptides wherein the chimeric fusion protein is designated IG2 comprising amino acid residues 2 to 174 of SEQ ID NO: 2.

3. The chimeric fusion protein of claim 1 or claim 2, wherein, when tested in an assay in a mouse model of IDDM, the chimeric fusion protein provides a greater reduction in the frequency of onset of diabetes than a control mixture containing equimolar amounts of each of the various individual peptide moieties comprised by the chimeric fusion protein, each of said individual peptide moieties and insulin chains not being covalently bound to any other of said individual peptide moieties and insulin chains in said mixture, wherein the assay is carried out by the repeated parenteral administration of a number of measured doses, each dose being of a predetermind molar quantity of said chimeric fusion protein in a pharmaceutically effective carrier or of said control mixture in the pharmaceutically effective carrier, the administration being at intervals of not less than twelve hours and not more than 72 hours between each of the doses.

4. The chimeric fusion protein of claim 3, wherein the mouse model of IDDM is the NOD mouse CYTOXAN induced diabetes model.

5. The chimeric fusion protein of claim 3, wherein the mouse model of IDDM is the NOD/SCID mouse adoptive transfer diabetes model.

6. The use of a polypeptide according to any of claims 1 and 2 in the preparation of a medicament for treating a patient selected from the group consisting of: patients predicted to be at risk of developing Type 1 diabetes and patients suffering from Type 1 diabetes, wherein the use comprises the repeated administration of a dose of a measured amount of the polypeptide of any one of claims I and 2.

7. The use according to claim 6 wherein the repeated administration comprises administration of at least two doses at an interval of not less than twelve hours and not more than 72 hours.

8. A chimeric fusion protein comprising an amino terminus and a carboxyl terminus, wherein the protein comprises insulin chain B and individual peptide moieties consisting of one or more GAD 65 peptides capable of eliciting a human T-cell response, wherein the insulin chain B and the one or more human GAD 65 peptides are covalently linked and the chimeric fusion protein is capable of eliciting a human T-cell response to insulin chain B and to one or more of the GAD 65 peptides wherein the GAD 65 peptide is selected from the group consisting of GAD 65 115-127 (amino acid residues 39-51 of SEQ ID NO:2), GAD 65 247-286 (amino acid residues 52-91 of SEQ ID NO:2), and GAD 65 473-519 (amino acid residues 92-138 of SEQ ID NO:2) and wherein the fusion protein does not comprise the GAD peptides selected from any of the following: GAD 65 residues 1-95; GAD 65 residues 475-484; GAD 65 residues 520-554 and GAD 65 residues 571-585.

9. A chimeric fusion protein according to claim 8, wherein the protein comprises in order, starting at the amino terminus, human GAD 65 peptides 115-127, 247-286, and 473-519.

10. A chimeric fusion protein according to claim 9, wherein the protein comprises in order, starting at the amino terminus, insulin B chain, insulin C chain and human GAD 65 peptides 115-127, 247-286, and 473-519.

## Patentansprüche

1. Chimäres Fusionsprotein, welches einen Aminoterminus und einen Carboxylterminus umfaßt, wobei das Protein die B-Kette von Insulin und einzelne Peptidreste, bestehend aus wenigstens einem GAD 65-Peptid, das in der Lage ist, eine Antwort von menschlichen T-Zellen auszulösen, umfaßt, wobei die B-Kette von Insulin und das wenigstens eine GAD 65-Peptid kovalent verknüpft sind und das chimäre Fusionsprotein in der Lage ist, eine Antwort von menschlichen T-Zellen auf die B-Kette von Insulin und auf jedes des wenigstens einen GAD 65-Peptids auszulösen, wobei das chimäre Fusionsprotein als IG1 bezeichnet wird und die Aminosäurereste 2 bis 154 von SEQ ID NO:1 umfaßt.

2. Chimäres Fusionsprotein, welches einen Aminoterminus und einen Carboxylterminus umfaßt, wobei das Protein die B-Kette von Insulin und einzelne Peptidreste, bestehend aus wenigstens einem GAD 65-Peptid, das in der Lage ist, eine Antwort von menschlichen T-Zellen auszulösen, umfaßt, wobei die B-Kette von Insulin und das wenigstens eine GAD 65-Peptid kovalent verknüpft sind und das chimäre Fusionsprotein in der Lage ist, eine Antwort von menschlichen T-Zellen auf die B-Kette von Insulin und auf jedes des wenigstens einen GAD 65-Peptids auszulösen, wobei das chimäre Fusionsprotein als IG2 bezeichnet wird und die Aminosäurereste 2 bis 174 von SEQ ID NO:2 umfaßt.

3. Chimäres Fusionsprotein nach Anspruch 1 oder Anspruch 2, wobei beim Testen in einem Assay in einem Maus-Modell von IDDM das chimäre Fusionsprotein eine stärkere Reduzierung der Häufigkeit des Beginns von Diabetes liefert als ein Kontrollgemisch, welches äquimolare Mengen von jedem der verschiedenen einzelnen Peptidreste, die das chimäre Fusionsprotein umfaßt, enthält, wobei keine(r) der einzelnen Peptidreste und Insulinketten mit irgendeinem (irgendeiner) anderen der einzelnen Peptidreste und Insulinketten in dem Gemisch kovalent verknüpft ist, wobei der Assay durch die wiederholte parenterale Verabreichung einer Anzahl bemessener Dosen durchgeführt wird, wobei jede Dosis aus einer vorbestimmten molaren Menge des chimären Fusionsproteins in einem pharmazeutisch wirksamen Träger oder des Kontrollgemischs in dem pharmazeutisch wirksamen Träger zusammengesetzt ist und die Verabreichung in intervallen von nicht weniger als zwölf Stunden und nicht mehr als 72 Stunden zwischen den einzelnen Dosen erfolgt.

4. Chimäres Fusionsprotein nach Anspruch 3, wobei das Maus-Modell von IDDM das CYTOXAN-induzierte Diabetesmodell der NOD-Maus ist.

5. Chimäres Fusionsprotein nach Anspruch 3, wobei das Maus-Modell von IDDM das Diabetesmodell der passiven Immunisierung der NOD/SCID-Maus ist.

6. Verwendung eines Polypeptids nach einem der Ansprüche 1 und 2 bei der Herstellung eines Medikaments zur Behandlung eines Patienten, ausgewählt aus der Gruppe, bestehend aus: Patienten, bei denen ein Risiko zur Entwicklung von Diabetes-Typ 1 vorhergesagt wird, und an Diabetes-Typ 1 leidenden Patienten, wobei die Verwendung die wiederholte Verabreichung einer Dosis einer bemessenen Menge des Polypeptids nach einem der Ansprüche 1 und 2 umfaßt.

7. Verwendung nach Anspruch 6, wobei die wiederholte Verabreichung die Verabreichung von wenigstens zwei Dosen in einem Intervall von nicht weniger als zwölf Stunden und nicht mehr als 72 Stunden umfaßt.

8. Chimäres Fusionsprotein, welches einen Aminoterminus und einen Carboxylterminus umfaßt, wobei das Protein die B-Kette von Insulin und einzelne Peptidreste, bestehend aus einem oder mehreren GAD 65-Peptiden, die in der Lage sind, eine Antwort menschlicher T-Zellen auszulösen, umfaßt, wobei die B-Kette von Insulin und das eine oder die mehreren GAD 65-Peptid(e) kovalent verknüpft sind und das chimäre Fusionsprotein in der Lage ist, eine Antwort menschlicher T-Zellen auf die B-Kette von Insulin und auf eines oder mehrere der GAD 65-Peptide auszulösen, wobei das GAD 65-Peptid aus der Gruppe ausgewählt ist, bestehend aus GAD 65 115-127 (Aminosäurereste 39-51 von SEQ ID NO:2), GAD 65 247-286 (Aminosäurereste 52-91 von SEQ ID NO:2) und GAD 65 473-519 (Aminosäurereste 92-138 von SEQ ID NO:2), und wobei das Fusionsprotein nicht die GAD-Peptide umfaßt, die unter den folgenden ausgewählt sind: GAD 65 Reste 1-95, GAD 65 Reste 475-484, GAD 65 Reste 520-554 und GAD 65 Reste 571-585.

9. Chimäres Fusionsprotein nach Anspruch 8, wobei das Protein in der Reihenfolge beginnend bei dem Aminoterminus die menschlichen GAD 65-Peptide 115-127, 247-286 und 473-519 umfaßt.

10. Chimäres Fusionsprotein nach Anspruch 9, wobei das Protein in der Reihenfolge beginnend bei dem Aminoterminus die B-Kette von Insulin, die C-Kette von Insulin und die menschlichen GAD 65-Peptide 115-127, 247-286 und 473-519 umfaßt.

## Revendications

1. Protéine de fusion chimérique comprenant une extrémité amino et une extrémité carboxyle, dans laquelle la protéine comprend une chaîne B d'insuline et des fractions peptidiques individuelles constituées par au moins un peptide GAD 65 capable d'induire une réponse des lymphocytes T humains, dans laquelle la chaîne B d'insuline et le au moins un peptide GAD 65 humain sont liés de manière covalente et la protéine de fusion chimérique est capable d'induire une réponse des lymphocytes T humains envers la chaîne B d'insuline et envers chacun du au moins un peptide GAD 65, la protéine de fusion chimérique étant appelée IG1 comprenant les résidus d'acides aminés 2 à 154 de SEQ. ID N° 1.

2. Protéine de fusion chimérique comprenant une extrémité amino et une extrémité carboxyle, dans laquelle la protéine comprend une chaîne B d'insuline et des fractions peptidiques individuelles constituées par au moins un peptide GAD 65 capable d'induire une réponse des lymphocytes T humains, dans laquelle la chaîne B d'insuline et le au moins un peptide GAD 65 humain sont liés de manière covalente et la protéine de fusion chimérique est capable d'induire une réponse des lymphocytes T humains envers la chaîne B d'insuline et envers chacun du au moins un peptide GAD 65, la protéine de fusion chimérique étant appelée IG2 comprenant les résidus d'acides aminés 2 à 174 de SEQ. ID N° 2.

3. Protéine de fusion chimérique selon la revendication 1 ou la revendication 2, dans laquelle, lors d'une analyse par dosage dans un modèle murin de DSID (diabète insulino-dépendant), la protéine de fusion chimérique permet d'obtenir une plus grande réduction de la fréquence de survenue du diabète qu'un mélange témoin contenant des quantités équimolaires de chacune des diverses fractions peptidiques individuelles constituant la protéine de fusion chimérique, chacune desdites fractions peptidiques individuelles et chaînes d'insuline n'étant liées de manière covalente à aucune autre desdites fractions peptidiques individuelles et chaînes d'insuline dans ledit mélange, dans laquelle le dosage est réalisé par administration répétée par voie parentérale d'un certain nombre de doses mesurées, chaque dose étant une quantité molaire prédéterminée de ladite protéine de fusion chimérique dans un véhicule efficace sur le plan pharmaceutique ou dudit mélange témoin dans le véhicule efficace sur le plan pharmaceutique, l'administration se faisant à des intervalles de pas moins de 12 heures et pas plus de 72 heures entre chacune des doses.

4. Protéine de fusion chimérique selon la revendication 3, dans laquelle le modèle murin de DSID est le modèle de diabète induit par CYTOXAN chez la souris NOD.

5. Protéine de fusion chimérique selon la revendication 3, dans laquelle le modèle murin de DSID est le modèle de diabète par transfert adoptif chez la souris NOD/SCID.

6. Utilisation d'un polypeptide selon l'une quelconque des revendications 1 et 2 dans la préparation d'un médicament pour le traitement d'un patient choisi dans le groupe constitué par : les patients supposés susceptibles de développer un diabète de type I et les patients souffrant de diabète de type I, dans laquelle l'utilisation comprend l'administration répétée d'une dose d'une quantité mesurée du polypeptide selon l'une quelconque des revendications 1 et 2.

7. Utilisation selon la revendication 6, dans laquelle l'administration répétée comprend l'administration d'au moins deux doses à un intervalle de pas moins de 12 heures et pas plus de 72 heures.

8. Protéine de fusion chimérique comprenant une extrémité amino et une extrémité carboxyle, dans laquelle la protéine comprend une chaîne B d'insuline et des fractions peptidiques individuelles constituées par un ou plusieurs peptides GAD 65 capables d'induire une réponse des lymphocytes T humains, dans laquelle la chaîne B d'insuline et les un ou plusieurs peptides GAD 65 humains sont liés de manière covalente et la protéine de fusion chimérique est capable d'induire une réponse des lymphocytes T humains envers la chaîne B d'insuline et envers un ou plusieurs des peptides GAD 65, dans laquelle le peptide GAD 65 est choisi dans le groupe constitué par GAD 65 115 à 127 (résidus d'acides aminés 39 à 51 de SEQ. ID N° 2), GAD 65 247 à 286 (résidus d'acides aminés 52 à 91 de SEQ. ID N° 2) et GAD 65 473 à 519 (résidus d'acides aminés 92 à 138 de SEQ. ID N° 2), et dans laquelle la protéine de fusion ne comprend pas les peptides GAD choisis parmi l'un quelconque des suivants : GAD 65 résidus 1 à 95 ; GAD 65 résidus 475 à 484 ; GAD 65 résidus 520 à 554 et GAD 65 résidus 571 à 585.

9. Protéine de fusion chimérique selon la revendication 8, dans laquelle la protéine comprend dans l'ordre, en partant de l'extrémité amino, les peptides GAD 65 humains 115 à 127, 247 à 286 et 473 à 519.

10. Protéine de fusion chimérique selon la revendication 9, dans laquelle la protéine comprend dans l'ordre, en partant de l'extrémité amino, une chaîne B d'insuline, une chaîne C d'insuline et les peptides GAD 65 humains 115 à 127, 247 à 286 et 473 à 519.
